# EUROPEAN PATENT SPECIFICATION

(11) **EP 1 214 106 B1**
(45) Date of publication and mention of the grant of the patent: **19.11.2003**
(21) Application number: 99945787.2
(22) Date of filing: 22.09.1999
(51) Int. Cl.: A61L 27/54, A61K 31/19, A61K 31/365

(54) **CARDIAC VALVE AND METHOD FOR PREPARING A BIOLOGICAL TISSUE**
HERZKLAPPE UND VERFAHREN ZU HERSTELLUNG EINES BIOLOGISCHEN GEWEBES
VALVULE CARDIAQUE ET PROCEDE DE PREPARATION D'UN TISSU BIOLOGIQUE

(43) Date of publication of application: 19.06.2002
(73) Proprietor: BAXTER INTERNATIONAL INC., Deerfield, Illinois 60015 (US)
(72) Inventor: TASIAUX, Nicole, B-1170 Bruxelles (BE); DELMOTTE, Yves, B-7332 Neufmaison (BE)
(74) Representative: Dee, Ian Mark
(86) International application number: BE9900121
(87) International publication number: WO01021228

(56) References cited:
- EP-A- 0 121 008
- WO-A-89/06945
- WO-A-92/19597
- US-A- 4 753 652
- US-A- 5 420 114
- US-A- 5 843 471
- PATENT ABSTRACTS OF JAPAN vol. 1999, no. 01, 29 January 1999 (1999-01-29) & JP 10 262564 A (SAKAI SANGYO KK), 6 October 1998 (1998-10-06)

## Description

This invention relates to a cardiac valve made from a biological or biocompatible tissue having a resistance to calcification.

Cardiac valves are known from documents US5476516, us 5002566 and WO89/06945, which are made from biological tissues stabilized by an aldehyde, e.g. a glutaraldehyde, and which have been complementarily treated with a polyol, such as polypropylene glycol, butanediol, pentanediol, etc., or with a solution containing an iron or tin salt, or with an aliphatic carboxylic acid, or with an ester of such acid. cardiac valves which have been treated as described in these documents have a more or less reduced calcification, but cannot suppress it totally.

In this invention, a family of particular compounds has been selected, providing an excellent calcification resistance. Further, in the case of a biological tissue, this selection of such compounds ensures that the tissue is stabilized and that its structure is preserved, both as regards proteins and lipids.

The cardiac valve according to the invention is made from a support associated to at least one compound having at least one ring of 6 carbon atoms with at least two hydroxyl groups, preferably at least three hydroxyl groups thereon.

Advantageously, the valve is at least partially made from a biological tissue and/or from a polymer or copolymer compound, particularly from a biopolymer compound, and/or from an at least partly cross-linked and biocompatible compound, said tissue and/or compound being associated to at least one compound having at least one ring of 6 carbon atoms with at least two hydroxyl groups, preferably at least three hydroxyl groups thereon.

Advantageously, the valve according to the invention has, at least at its surface, a polymer or copolymer or an at least partly cross-linked compound, associated to at least one compound having at least one ring of 6 carbon atoms with at least two hydroxyl groups, preferably at least three hydroxyl groups thereon.

According to a preferred embodiment, the valve consists of a tissue, particularly of a biological tissue, stabilized by a polymer or copolymer or an at least partly cross-linked compound, said compound, polymer or copolymer advantageously forming a network, said compound, polymer or copolymer being associated to at least one compound having at least one ring of 6 carbon atoms with at least two hydroxyl groups, preferably at least three hydroxyl groups thereon. An appropriate biological tissue may be, for example, a biological tissue removed from the heart of an animal, or from the aortic valve of an animal, or from the pericardium of an animal, said tissue being advantageously stabilized by a cross-linkable compound, such as an aldehyde, particularly a glutaraldehyde, wherein the aldehyde (particularly the glutaraldehyde) which is at least at the surface of the tissue or in the proximity thereof is at least partially associated to a compound having at least one ring of 6 carbon atoms with at least two hydroxyl groups, preferably at least three hydroxyl groups thereon.

It has been noted that the use of such compounds provided the cardiac valve with calcification resistance properties, with good mechanical properties, with easy rinse and/or sterilization features and, in the case of a biological tissue, with the possibility to preserve the structure thereof, both as regards proteins and lipids (phospholipids).

In accordance with possible embodiments, the valve is made from biopolymers, the composition of which can contain one or more substances selected from the group consisting of polylactic compounds, polyglycolic compounds, modified hyaluroic acid , collagen, fibrin, fibronectin, etc., and mixtures thereof.

Advantageously, the compound having at least one ring of 6 carbon atoms with at least two hydroxyl groups, preferably at least three hydroxyl groups thereon, is selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and sal ts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acids, and mixtures thereof.

By way of example, the following compounds may be mentioned:
* gallotannins, particularly
   - tannins with formula 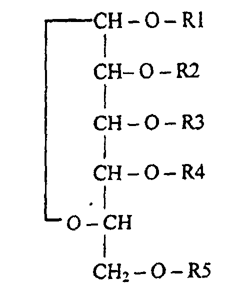 where R1 = R2 = R3 = R4 = the rest of gallic or digallic acid, and R5 = H; or
      where R1, R2, R3, R4 and R5 are selected from the rest of gallic or digallic acid or
      where R1 = R3 = R4 = the rest of gallic acid, R2 = H and 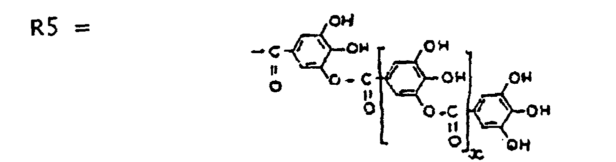 where x = 0, 1, 2, 3 or 4
   - tannins with formula 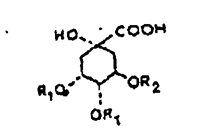
   where R1 = the rest of gallic or digallic acid, and 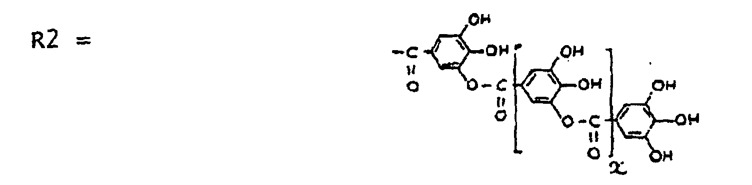 where x = 0, 1, 2, 3 or 4, or
* ellagitannins, particularly tannins with formula 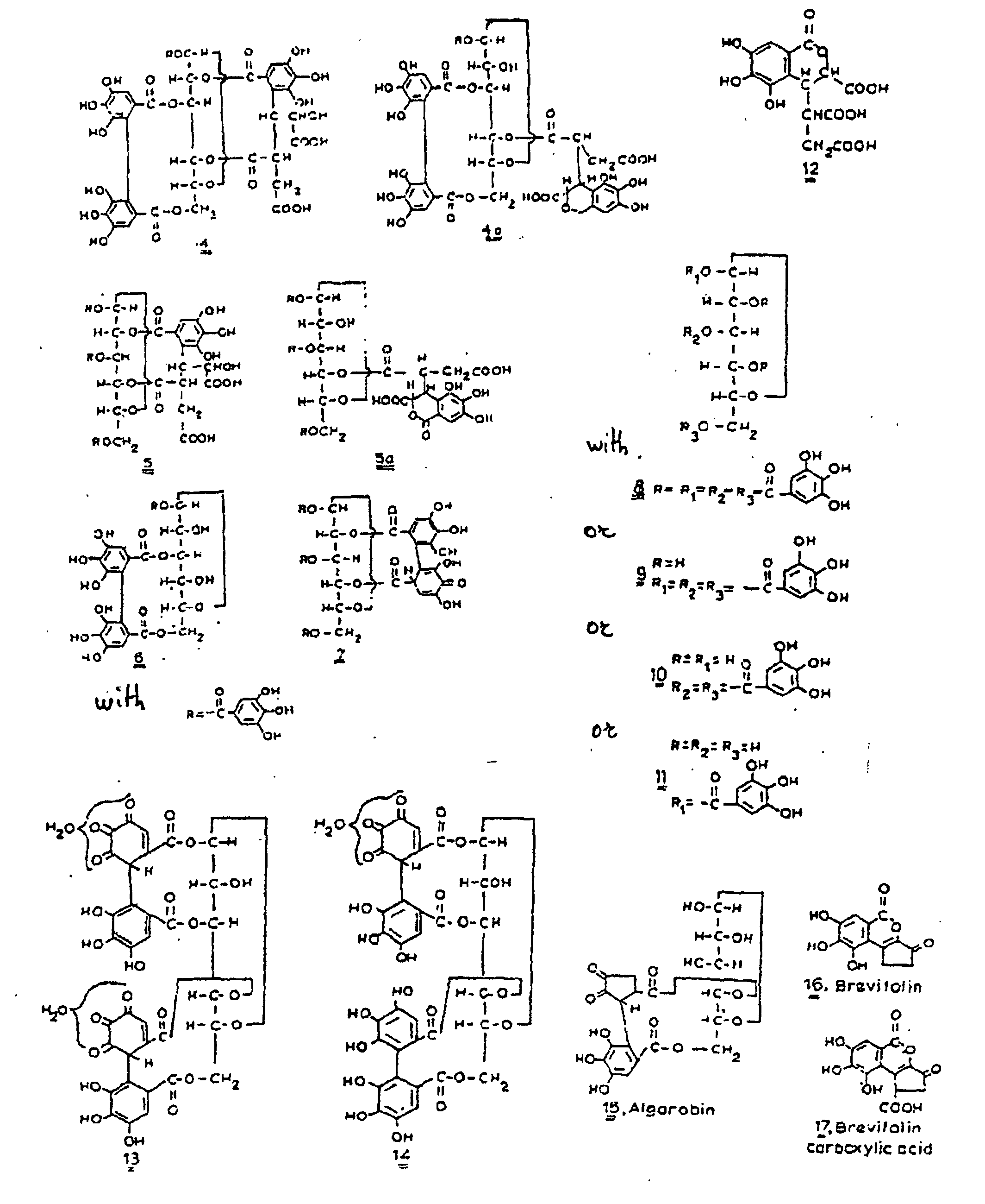 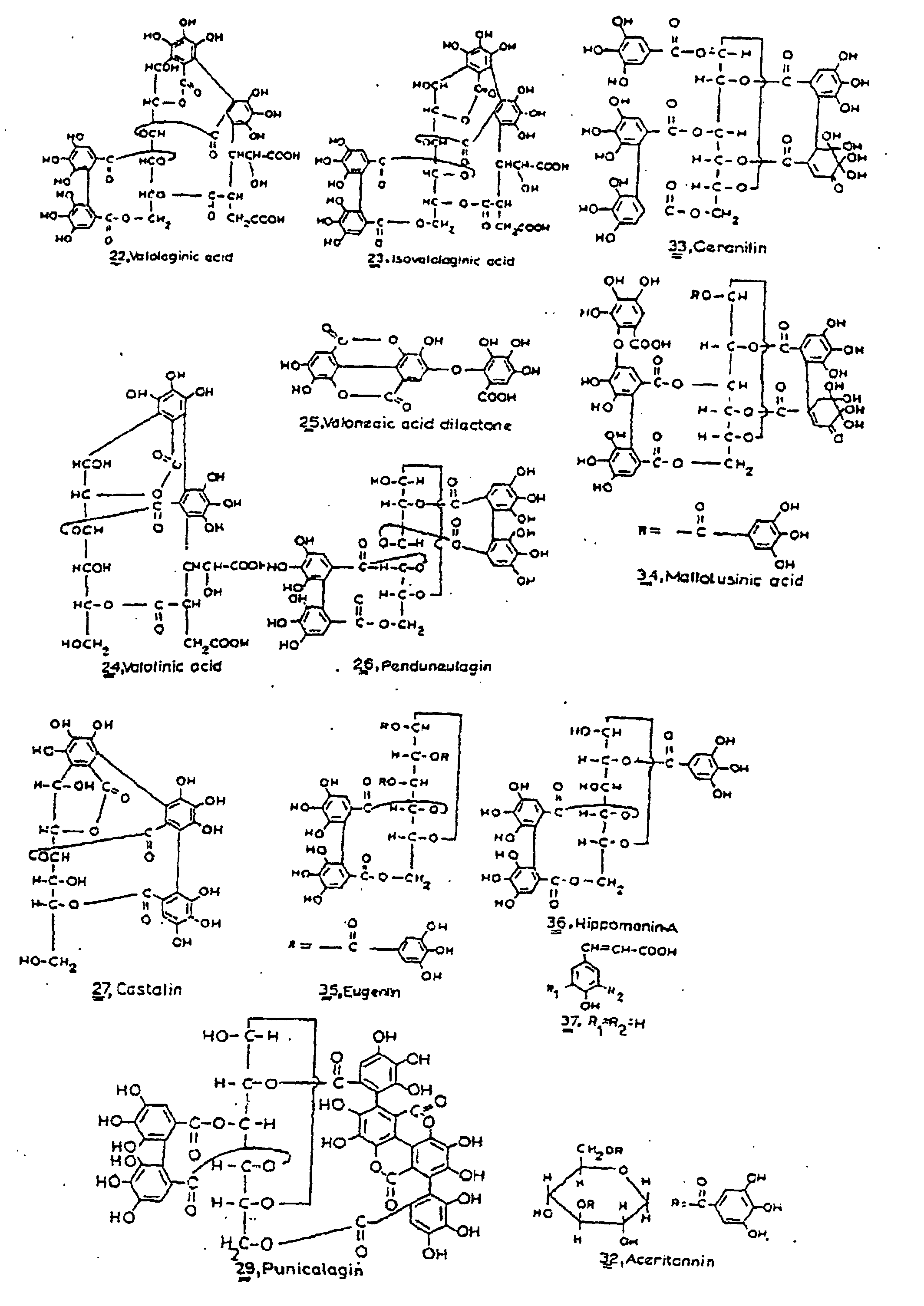 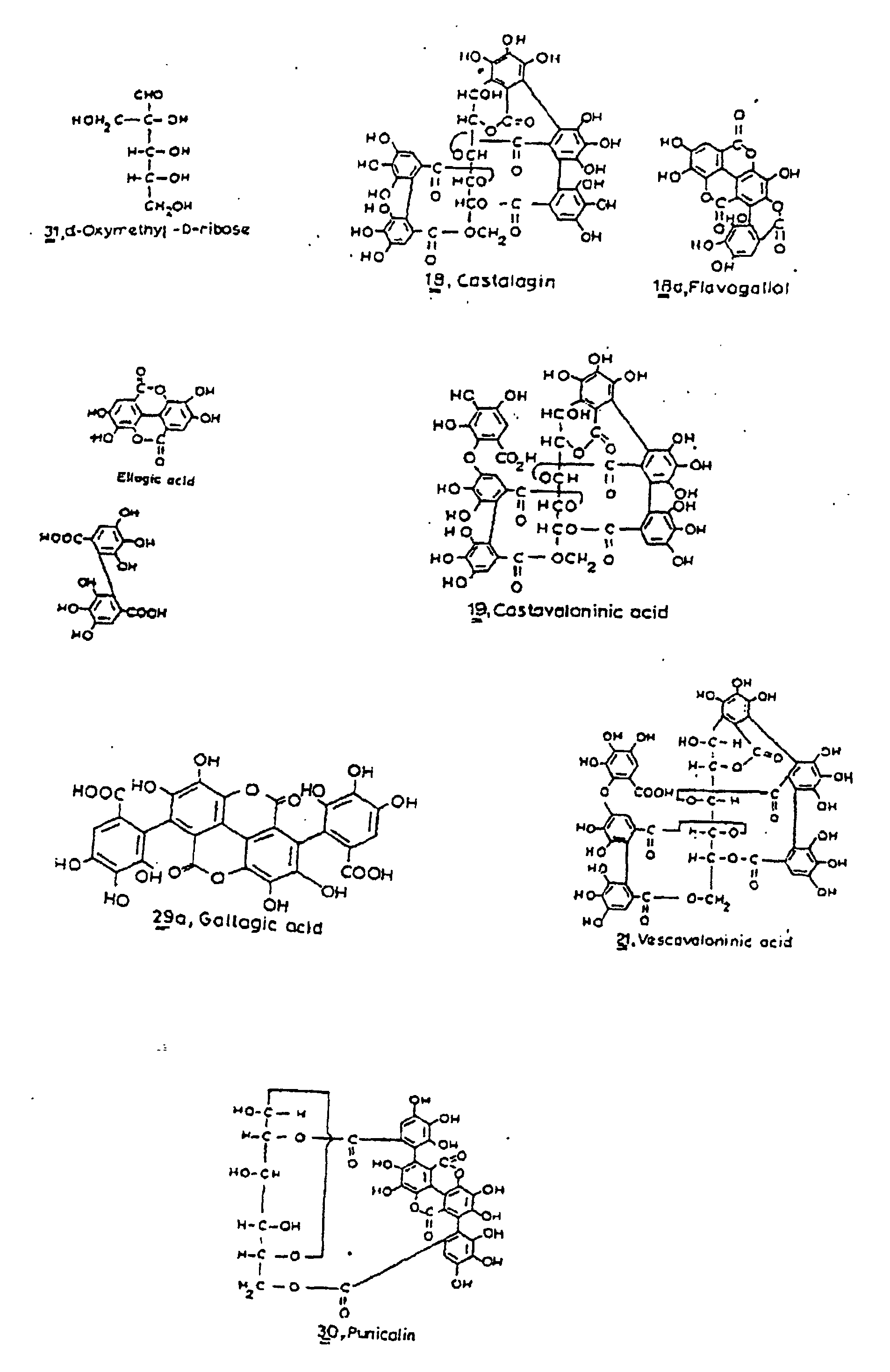
* condensed tannins, particularly tannins with formula 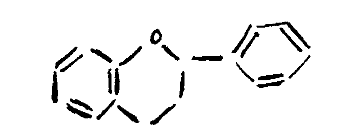 With two, preferably three hydroxyl groups 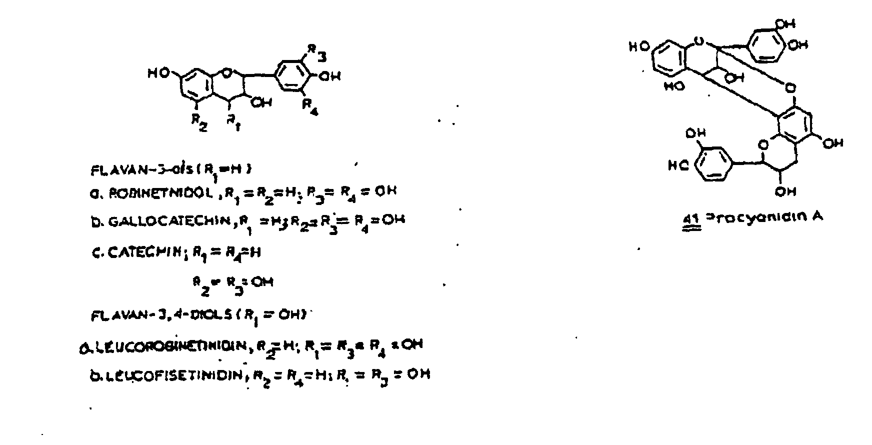 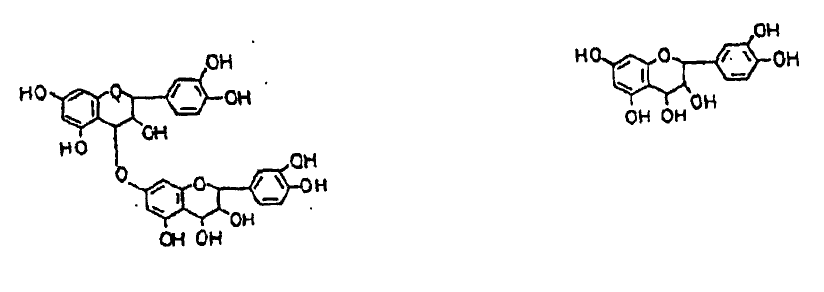 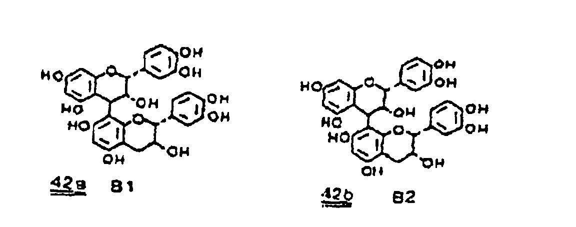 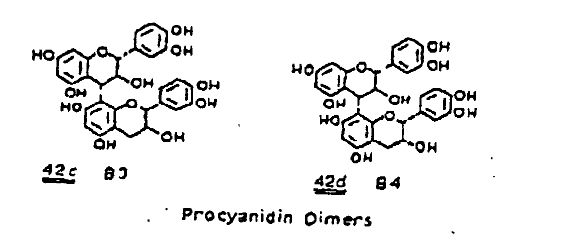 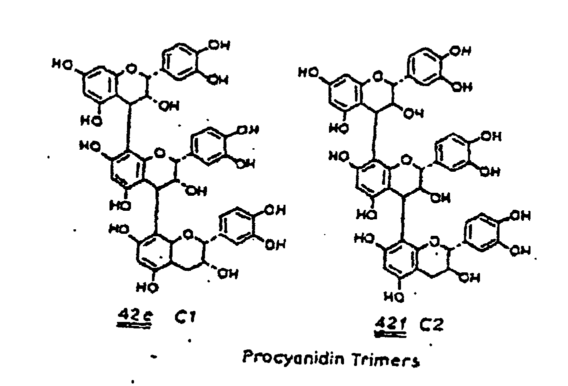 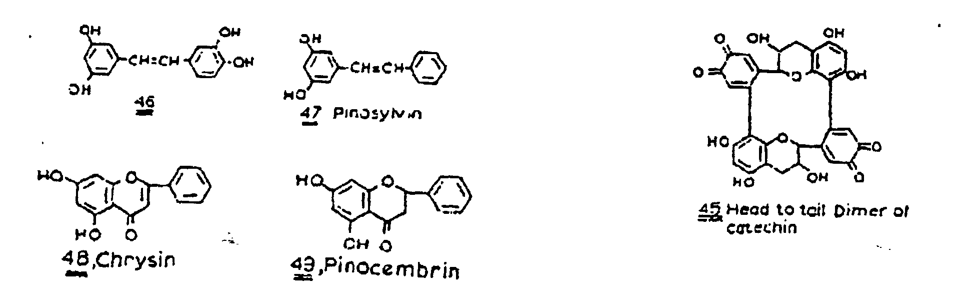 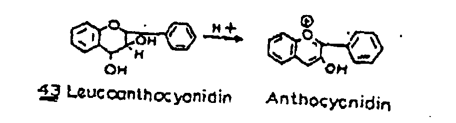 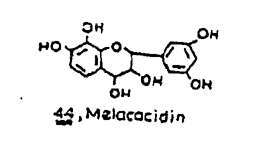 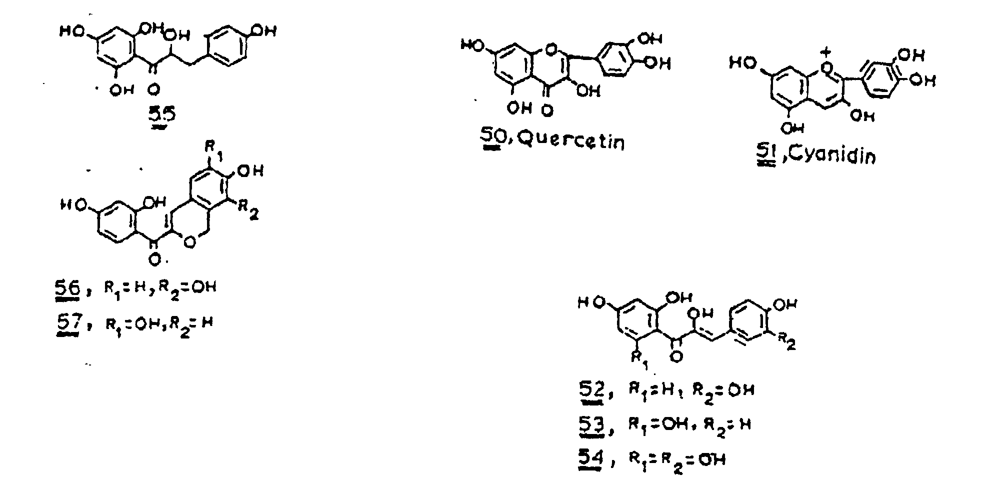 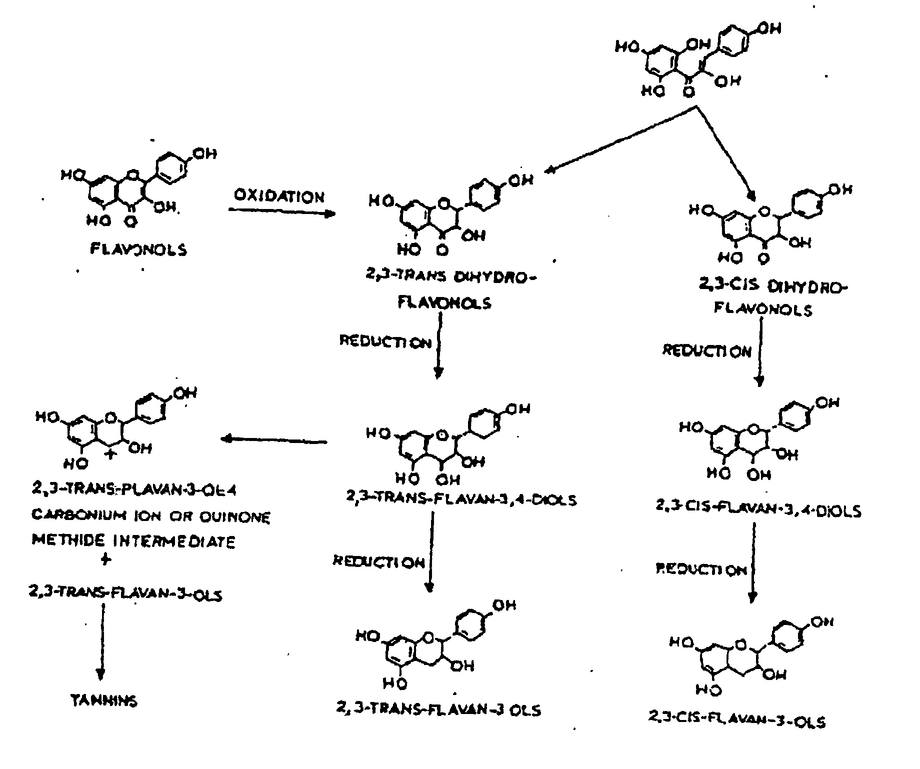
* gallic acid
* digallic acid
* quinic acid
* 5-dehydroquinic acid
* shikimic acid
* 5-dehydroshikimic acid
* vescalin 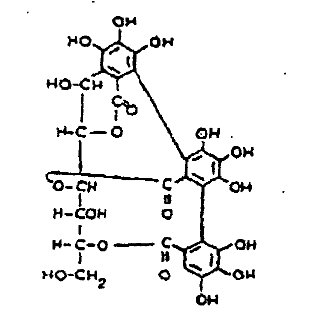
* vascalagin 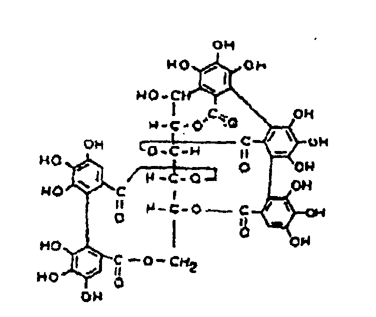
* salts and esters of these acids, particularly aliphatic, cycloaliphatic, aromatic, phosphoric esters, polyesters, etc.
* hydrolysis products of salts and esters of the above compounds, particularly aliphatic, cycloaliphatic, aromatic, phosphoric esters, polyesters, etc.
* condensation products of aldehyde, such as formaldehyde, glutaraldehyde, etc., with tannins, especially tannic acids of the general formula described hereabove
or with vescalin and/or vascalagin. Preferred condensation products are condensation products of aldehyde, formaldehyde and/or glutaraldehyde with tannins, tannic acids, quinic acid, dehydroquinic acid, gallic acid, digallic acid, shikimic acid, dehydroshikimic acid, vescalin and/or vascalagin. Advantageously, at least two moles of tannins or tannic acids are used per mole of aldehyde. Preferably the aldehyde function of the aldehyde compounds are completely or substantially completely condensed with tannic acids or tannins.

The rest of gallic or digallic acid is intended as the radical obtained after removal of an OH group from the carboxylic function, i.e.
- (CO)-(C₆H₅O₃) for gallic acid, and
- (CO)-(C₆H₄O₂)-O-(CO)-(C₆H₅O₃) for digallic acid.

Preferably, in the cardiac valve according to the invention, the compound having at least one ring of 6 carbon atoms with at least two hydroxyl groups, preferably at least three hydroxyl groups thereon is selected from the group consisting of hydrolyzable tannins, salts of these acids, esters of these acids, hydrolysis products of said salts and esters, vescalin, vascalagin, hydrolysis products of vescalin or vascalagin, esters and salts of vescalin and vascalagin, condensation product of an aldehyde with said tannins or tannic acid and mixtures thereof.

More advantageously, the compound having at least one ring of 6 carbon atoms with at least two hydroxyl groups, preferably at least three hydroxyl groups thereon is selected from the group consisting of the tannic acid with formula 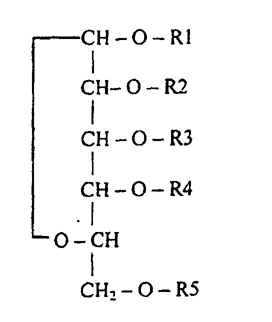 where R1, R2, R3, R4 and R5: the rest of gallic acid or digallic acid; salts and esters of these tannic acids; quinic acid; dehydroquinic acid; esters and salts of qui ni c acid and of dehydroqui ni c acid; gallic acid; digallic acid; esters and salts of gallic acid and of digallic acid; hydrolysis products of esters and salts of these acids, vescalin, vascalagin, hydrolysis products of vescalin or vascalagin, esters and salts of vescalin and vascalagin, condensation product of an aldehyde with said tannins or tannic acid (especially the tannins and tannic acids disclosed hereabove) and mixtures thereof.

More advantageously, the cardiac valve according to the invention has, at its surface, an advantageously substantially continuous and substantially homogeneous layer, associated to or containing a compound selected from the group consisting of tannic acids with formula 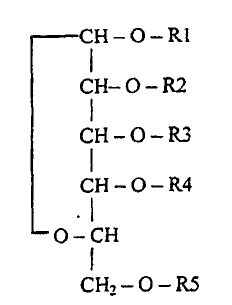 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of these salts and esters, vescalin, vascalagin, hydrolysis products of vescalin or vascalagin, esters and salts of vescalin and vascalagin, condensation product of an aldehyde with said tannins or tannic acid (preferably the tannins and tannic acids as listed hereabove) and mixtures thereof.

According to a particular embodiment, the valve has the form of a body associated, both at its surface and inside it, to one or more compounds selected from the group consisting of acids with formula 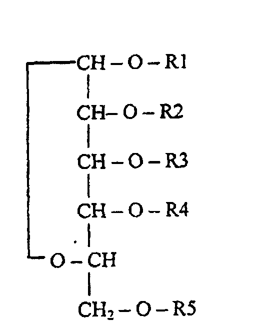 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and salts of gallic acid and of digallic acid, hydrolysis products of these salts and esters, vescalin, vascalagin, hydrolysis products of vescalin or vascalagin, esters and salts of vescalin and vascalagin, condensation product of an aldehyde with said tannins or tannic acid and mixtures thereof.

In the case of a biological tissue stabilized by an aldehyde, the latter allows to create bonds between the collagen of the tissue and inside the tissue.

The invention also rel ates to the use of a body or tissue containing at least one biological compound and/or at least one polymer or copolymer compound and/or at least one partially cross-linked and biocompatible compound, said compound being associated at least partially to a compound having at least one ring of 6 carbon atoms with at least two hydroxyl groups, preferably at least three hydroxyl groups thereon, for preparing an animal or human implant, particularly a cardiac implant, such as a cardiac valve, or vessels, ligaments, tendons, tracheas, membranes, esophagi, etc.

Advantageously, said compound having at least one ring of 6 carbon atoms with at least two hydroxyl groups, preferably at least three hydroxyl groups thereon is a compound as described hereinbefore as regards the cardiac valve according to the invention.

In accordance with a particular embodiment, the tissue in use is a biological tissue stabilized by an aldehyde, particularly by a glutaraldehyde, wherein the aldehyde (particularly the glutaraldehyde) at the surface of the tissue or in the proximity thereof is associated to at least one compound having at least one ring of 6 carbon atoms with at least two hydroxyl groups, preferably at least three hydroxyl groups thereon.

Preferably, the biological tissue used as an implant is stabilized by an aldehyde associated to a compound as described hereinbefore as regards cardiac valves.

More advantageously, the tissue or body used as an implant is associated at least at its surface to a compound selected from the group consisting of the tannic acid with formula 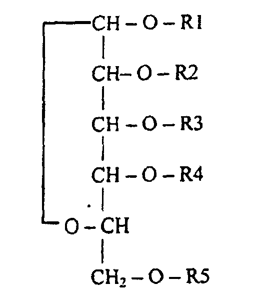 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of esters and salts of these acids, vescalin, vascalagin, hydrolysis products of vescalin or vascalagin, esters and salts of vescalin and vascalagin, condensation product of an aldehyde with said tannins or tannic acid and mixtures thereof.

More advantageously, the tissue or body, particularly the biological tissue used as an implant according to the invention has, at its surface, a substantially continuous and substantially homogeneous layer, containing or associated to one or more compounds selected from the group consisting of tannic acids with formula 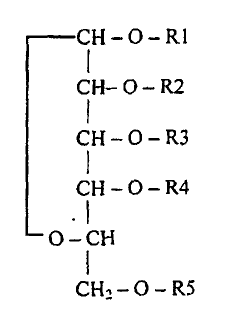 where R1, R2, R3, R4 and R5: the rest of gallic acid or digallic acid; salts and esters of these tannic acids; quinic acid; dehydroquinic acid; esters and salts of quinic acid and of dehydroqui ni c acid; gallic acid; digallic acid; esters and salts of gallic acid and of digallic acid; hydrolysis products of these salts and esters, vescalin, vascalagin, hydrolysis products of vescalin or vascalagin, esters and salts of vescalin and vascalagin, condensation product of an aldehyde with said tannins or tannic acid, and mixtures thereof.

According to a particular embodiment, the advantageously biological tissue used as an implant contains, both at its surface and inside it, a compound selected from the group consisting of tannic acids with formula: 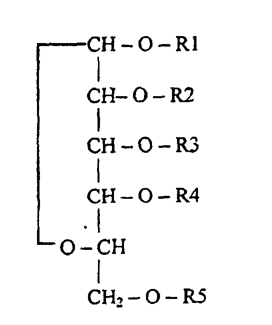 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of these salts and esters, vescalin, vascalagin, hydrolysis products of vescalin or vascalagin, esters and salts of vescalin and vascalagin, condensation product of an aldehyde with said tannins or tannic acid and mixtures thereof.
In the case of a biological tissue, the presence of such a compound inside the body or tissue allows to create bonds between the collagen of the tissue and, for instance, aldehyde inside the tissue or body.

The invention further relates to a method for preparing a cardiac valve or a tissue or body used as an implant,
wherein this implant is treated with a solution containing a compound having at least one ring of 6 carbon atoms with at least two hydroxyl groups, preferably at least three hydroxyl groups thereon, or wherein said implant is at least partially prepared from a polymer or copolymer compound or from a cross-linkable biocompatible compound at least partially treated or mixed with a compound having at least one ring of 6 carbon atoms with at least two hydroxyl groups, preferably at least three hydroxyl groups thereon, and
wherein, possibly after a rinsing and/or washing step, the implant is further sterilized and/or treated aseptically. Aseptic treatment of the implant is intended, amongst other things, as aseptic cleaning of an advantageously sterilized implant, rinsing with water, e.g. sterile water for injections, removal of fragments or residues of tissue in a sterile room, implant shaping, etc.

As compound having at least one ring of 6 carbon atoms with at least two hydroxyl groups, preferably at least three hydroxyl groups thereon, it is advantageous to use a compound selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid and mixtures thereof.

Preferably, as compound having at least one ring of 6 carbon atoms with at least two hydroxyl groups, preferably at least three hydroxyl groups thereon, a tannic acid, a salt of this acid, an ester of this acid, or a hydrolysis product of said salt or ester is selected.

Particularly, as a compound having at least one ring of 6 carbon atoms with at least two hydroxyl groups, preferably at least three hydroxyl groups thereon, a compound is used which is selected from the group consisting of : the tannic acid with formula 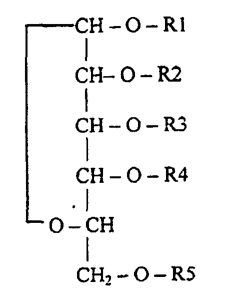 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of these salts and esters, vescalin, vascalagin, hydrolysis products of vescalin or vascalagin, esters and salts of vescalin and vascalagin, condensation product of an aldehyde with said tannins or tannic acid, and mixtures thereof.

Advantageously, the treatment is carried out for obtaining, at the surface of the implant, a layer containing or associated to a compound selected from the group consisting of : the tannic acid with formula 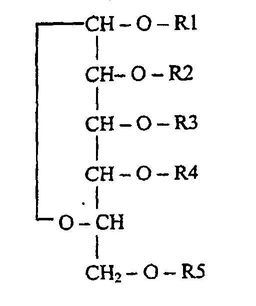 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of esters and salts of these acids vescalin, vascalagin, hydrolysis products of vescalin or vascalagin, esters and salts of vescalin and vascalagin, condensation product of an aldehyde with said tannins or tannic acids and mixtures thereof.

Particularly, the treatment is carried out for obtai ni ng an implant which contains both at its surface and inside its tissue, a compound selected from the group consisting of : the tannic acid with formula 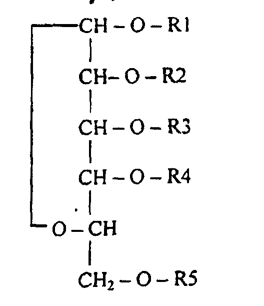 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of these salts and esters vescalin, vascalagin, hydrolysis products of vescalin or vascalagin, esters and salts of vescalin and vascalagin, condensation product of an aldehyde with said tannins or tannic acids and mixtures thereof.

For instance, the implant is treated with a solution containing a compound having at least one ring of 6 carbon atoms with at least two hydroxyl groups, preferably at least three hydroxyl groups thereon, and at least one solvent having at least one hydroxyl function, calcium-free water or water containing less than about 100 mg of calcium per liter.

According to an advantageous embodiment, the implant or tissue is treated with a solution containing a compound having at least one ring of 6 carbon atoms with at least two hydroxyl groups, preferably at least three hydroxyl groups thereon, said solution having a pH of 3 to 9, particularly of 5.5 to 7.5. According to a possible embodiment, the content of compound(s) having at least one ring of 6 carbon atoms with at least two hydroxyl groups, preferably at least three hydroxyl groups thereon, is of 0.1 to 10% by weight, preferably of 0.5 to 5% by weight, particularly of 1 to 3% by weight. This treatment step is advantageously executed at a temperature below 25°C, e.g. a temperature of 0 to 25°C, advantageously at a temperature of 0 to 8°C, preferably at about 4°C.

After this treatment step, or during this treatment step, the implant or valve or tissue is advantageously submitted to a photooxidation step. Photooxidation is performed, for instance, by exposing the valve or tissue to the rays of a lamp, e.g. a halogen lamp or a lamp emitting to light with a wave length of 400 - 800 nano meter. This treatment step is possibly executed with oxygen being added, e.g. by bubbling the treatment solution with air. This photooxidation step seems to be useful to provide bonds between collagen molecules of the tissue and/or between collagen molecules and tannic acid. Irradiation may possibly occur after transplantation, e.g. by means of an endoscope.

when a tissue of biological origin is used, this tissue is advantageously stabilized by an aldehyde, particularly a glutaraldehyde. stabilization is performed, for instance, by means of an advantageously aqueous solution containing 0.1 to 10% by weight, advantageously 0.2 to 5% by weight, preferably 0.3 to 1% by weight of aldehyde. Advantageously, aldehyde is glutaraldehyde. Stabilization advantageously occurs at a temperature below 25°C, e.g. a temperature of 0 to 25°C, advantageously a temperature of 0 to 8°C, preferably at about 4°C.

The implant so treated is advantageously aseptized and/o r sterilized. Sterilization may be performed by immersion in a glutaraldehyde and/or formaldehyde solution. For instance, sterilization is performed by means of an aqueous solution containing 0.6% by weight of glutaraldehyde, e.g. a solution buffered at a pH of 7 - 7.5. sterilization is performed, for instance at a temperature of 0 to 50°C, particularly at a temperature of about 4°C, or at a temperature of about 37°C.

Sterilization may be performed by other treatments, e.g. gamma irradiation.

The invention further relates to the use of at least one compound selected from the group consisting of: tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin esters and salts of vescalin and vascalagin, condensation product of an aldehyde with said tannins or tannic acid and mixtures thereof in the manufacture of a pharmaceutical composition containing an effective amount of paid at least one compound for treating or preventing calcification in a blood circuit.

Advantageously, the preparation contains, as an agent against calcification, particularly of a cardiac valve and/or of an implant in contact with blood, an effective amount of at least one compound selected from the group consisting of : the tannic acid with formula 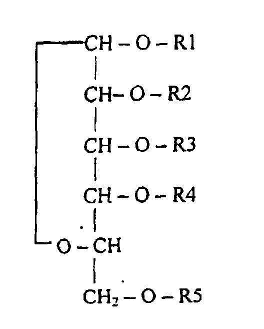 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of esters and salts of these acids, vescalin, vascalagin, hydrolysis products of vescalin or vascalagin, esters and salts of vescalin and vascalagin, and mixtures thereof.

Preferably, the preparation contains, as an agent against calcification, an effective amount of at least one compound selected from the group consisting of : tannic acids with formula 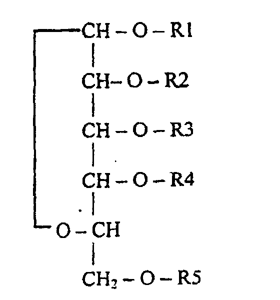 where R1, R2, R3, R4 and R5: the rest of gallic acid or digallic acid; salts and esters of these tannic acids; hydrolysis products of these salts and esters, vescalin, vascalagin, hydrolysis products of vescalin or vascalagin, esters and salts of vescalin and vascalagin, and mixtures thereof.

Although the preparation according to the invention may be in the (intravenously) injectable form, e.g. of a blood bag, particularly containing a tannic acid, in the liquid or solid oral form, in the suppository form, in the form of a cream to be applied on the skin, or in the form of patches to be applied on the skin, oral forms are preferred. Among oral forms, long release preparations are preferred.

Solid oral forms are, for example, capsules, matrices, pellets, pastilles, chewing-gums, tablets, etc. These forms may be of the substantially immediate release type and/or of the substantially constant release type. In the case of matrices, matrices controlling the liberation or release of the active compound are advantageously used. In the case of capsules or pellets or tablets, coverings or coatings, advantageously a succession of coatings are advantageously used to control the release of the compound. Particularly, at least one coating is an enteric coating. For example, the active compound is mixed with a wetting agent and possibly with a pharmacological excipient, the active compound is then transformed into pellets, pastilles, granules, etc. said pellets, pastilles, granules, etc. being coated with a film, or a microporous membrane, containing, for instance, a pharmacological excipient, and a film forming mixture containing an acrylic and/or methacrylic polymer or copol ymer which is insoluble in the organism and a polymer or non polymer substance (preferably a non acrylic and non methacrylic substance) which is insoluble in the acid gastric environment but is soluble in the intestine.

solid oral forms contain, for instance, an unitary dose of 10 mg to 5 g, advantageously 20 mg to 1 g, preferably 50 mg to 500 mg of the active principle, or of a mixture of active principles, e.g. a dose of 100 mg , 200 mg , 250 mg , 400 mg .

The composition or preparation according to the invention is suitable, for instance, for patients who have had a cardiac valve transplant, particularly involving the cardiac valve according to the invention.

The composition or preparation may also be administered to an animal, e.g. a pig, whose pericardium is to be removed to make cardiac valves for transplantation.

The composition or preparation containing possibly further active agent(s), is also suitable for treating or preventing diseases involving calcification, such as urolethiasis, artherosclerosis, hydatic diseases of the liver, cerebral or brain calcification, rheumatoid arthritis, SLE (systemic lupus erythematosus), calcinosis of the hand, carcinome, arterial plaque, tumor calcification, Dystrophic calcification, dental plaque, calculus and other diseases involving calcification which are listed in Arch Pathol Lab Med, Vol 107, July 1983, Calcific Diseases A concept by Anderson, pages 341 to 347.

The composition or preparation of the invention is also suitable in the treatment of calculus, such as kidney calculus, urether calculus, etc. by means of ultrasound(s) or litotripsis. The composition or preparation is given to a patient before his treatment by ultrasound(s) or litotripsis, so that the treatment has to be less severe. For example, during a few days, preferably at least one week before the treatment with ultrasound(s), the patient receives several doses of the composition or preparation. After such a treatment, the preparation or composition is advantageously administered for preventing or reducing the formation of calculus.

The composition or preparation of the invention can also take the form of a tooth pasta and/or buccal solution, such as a rinsing buccal solution.

The composition or preparation may also contain one or more further supplementary active principles. For example, the solid oral form may contain an agent against cholesterol ,particularly Zocor, Mevacor, a mixture of campestanol and sitostanol, etc.

The invention relates also to an implantable support intended to be in contact with a biological medium, especially with a human or animal medium, such as an implantable support, advantageously of a biological implantable support and/or an implantable support containing a polymer or copolymer compound, and/or an implantable support containing an at least partly cross-linked and biocompatible compound, said support being associated to at least one compound having at least one ring of 6 carbon atoms with at least two hydroxyl groups, preferably at least three hydroxyl groups thereon.

Examples of such support are: artificial heart or part thereof, artificial kidney or part thereof, pumps, micro pumps, insulin delivering pumps, bioreactors, stents, catheters, tubes, artificial veins, valves, polyurethane valves, sensors, fibrin membrane, power cells, pace makers, identification chips such as for dogs, chargers of power cells, and any other devices intended to be in contact with a biological medium, such as a biological fluid.

Advantageously, the support of the invention has, at least at one of its surface, one or more compounds having at least one ring of 6 carbon atoms with at least two hydroxyl groups, preferably at least three hydroxyl groups thereon, said compound/s being advantageously associated to the polymer or copolymer compound or to the at least partially cross-linked biocompatible compound.

Preferably, the support has the form of a biological tissue, stabilized at least partially by a polymer, copolymer or an at least partially cross-linked biocompatible compound, associated to at least one compound having at least one ring of 6 carbon atoms with at least two hydroxyl groups, preferably at least three hydroxyl groups thereon.

For example, the biological tissue is stabilized at least partially by an aldehyde, the aldehyde which is at least at the surface of the tissue or in the proximity thereof being at least partially associated to a compound having at least one ring of 6 carbon atoms with at least two hydroxyl groups, preferably at least three hydroxyl groups thereon.

Preferably, the compound having at least one ring of 6 carbon atoms with at least two hydroxyl groups, preferably at least three hydroxyl groups thereon, is selected from the group consisting of tannins, tannic acids, salts of tannic acids, esters of tannic acids, hydrolysis products of salts and esters of tannic acids and tannins, quinic acid, dehydroqui ni c acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid, and mixtures thereof.

Most preferably, the compound having at least one ring of 6 carbon atoms with at least two hydroxyl groups, preferably at least three hydroxyl groups thereon is selected from the group consisting of hydrolyzable tannic acids, salts of these acids, esters of these acids, hydrolysis products of said salts and esters, vescalin, vascalagin, hydrolysis products of vescalin or vascalagin, esters and salts of vescalin and vascalagin, condensation product of an aldehyde with said tannins or tannic acid, and mixtures thereof.

Typically, the compound having at least one ring of 6 carbon atoms with at least two hydroxyl groups, preferably at least three hydroxyl groups thereon is selected from the group consisting of the tannic acids with formula 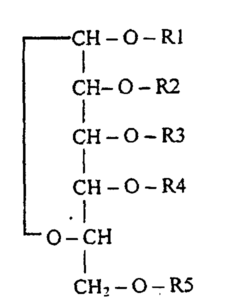 where R1, R2, R3 , R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of esters and salts of these acids, vescalin, vascalagin, hydrolysis products of vescalin or vascalagin, esters and salts of vescalin and vascalagin, condensation product of an aldehyde with said tannins or tannic acid and mixtures thereof.

According to an embodiment, the support has, at its surface in contact with the biological medium, a layer containing at least one compound selected from the group consisting of tannic acids with formula 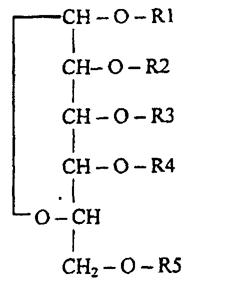 where R1, R2, R3, R4 and R5: the rest of gallic acid or digallic acid; salts and esters of these tannic acids; quinic acid; dehydroquinic acid; esters and salts of quinic acid and of dehydroquinic acid; gallic acid; digallic acid; esters and salts of gallic acid and of digallic acid; hydrolysis products of these salts and esters, vescalin, vascalagin, hydrolysis products of vescalin or vascalagin, esters and salts of vescalin and vascalagin, condensation product of an aldehyde with said tannins or tannic acids, and mixtures thereof.

However, preferably, the support has the form of a body having, both at its surface and inside it, a compound selected from the group consisting of compounds with formula 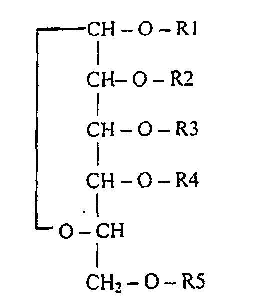 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and salts of gallic acid and of digallic acid, hydrolysis products of these salts and esters, vescalin, vascalagin, hydrolysis products of vescalin or vascalagin, esters and salts of vescalin and vascalagin, condensation product of an aldehyde with said tannins or tannic acids, and mixtures thereof.

still a further subject matter of the invention, is an aqueous stabilizing composition for a support selected from the group consisting of support to be in contact with a biological medium (such as a biological fluid, for example blood), implantable support, biological support, animal tissue, and human tissue, said composition containing:- at least an aldehyde in mixture with a compound selected from the group consisting of compounds with formula 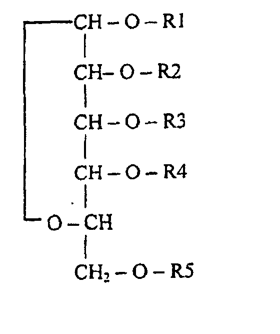 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and salts of gallic acid and of digallic acid, hydrolysis products of these salts and esters, vescalin, vascalagin, hydrolysis products of vescalin or vascalagin, esters and salts of vescalin and vascalagin, condensation product of an aldehyde with said tannins or tannic acids, and mixtures thereof, or - a compound selected from the group consisting of compounds with formula 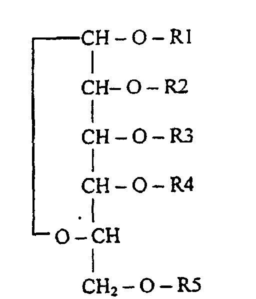 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and sal ts of gallic acid and of digallic acid, hydrolysis products of these salts and esters, vescalin, vascalagin, hydrolysis products of vescalin or vascalagin, esters and salts of vescalin and vascalagin, in mixture with a condensation product of an aldehyde with the above mentioned tannins or tannic acids.

Advantageously, the pH of the composition is comprised between 3 to 9, advantageously between 5.5 and 7.5, preferably about 7.

According to an embodiment, the composition contains up to 10%, advantageously less than 5%, preferably less than 2.5% by weight of a first compound selected from the group consisting of compounds with formula 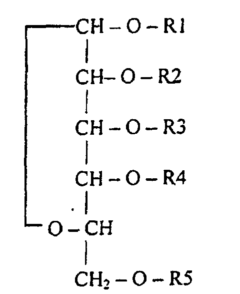 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and salts of gallic acid and of digallic acid, hydrolysis products of these salts and esters, vescalin, vascalagin, hydrolysis products of vescalin or vascalagin, esters and salts of vescalin and vascalagin, and mixtures thereof, and up to 10%, advantageously less than 5%, preferably less than 2.5% by weight of an aldehyde and/or a condensation product of an aldehyde with said tannins or tannic acids.

Preferably, the composition contains a phosphate buffer.

In the stabilizing composition of the invention, the weight ratio first compound/aldehyde is advantageously comprised between 1:10 and 10:1, preferably 1:5 and 5:1.

The stabilizing composition of the invention can be a ready-to-use composition, but is preferably marketed as a kit comprising a first vial containing the first compound as an aqueous solution, but preferably in the form of a powder, said first vial being substantially free of aldehyde, and a second vial containing an aqueous solution containing aldehyde (said vial being free or substantially free of tannic acid or tannin), the content of the said two vials having to be mixed together so as to prepare the stabilizing composition. Advantageously the second vial contains at least partly the phosphate buffer. The water used is advantageously sterile water, preferably pyrogen free water.

The kit of the invention for preparing a stabilizing composition of the invention comprises advantageously a first bottle containing, as a powder or in an aqueous solution (preferably as a powder), a first compound selected from the group consisting of compounds with formula 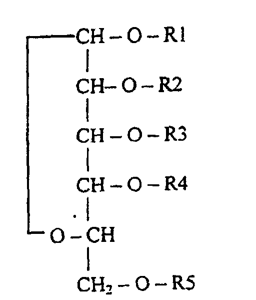 where R1, R2, R3, R4 and R5: the rest of gallic acid or digallic acid, salts and esters of these tannic acids, quinic acid, dehydroquinic acid, esters and salts of quinic acid and of dehydroquinic acid, gallic acid, digallic acid, esters and salts of gallic acid and of digallic acid, hydrolysis products of these salts and esters, vescalin, vascalagin, hydrolysis products of vescalin or vascalagin, esters and salts of vescalin and vascalagin, (said first vial being preferably free or substantially free of aldehyde) and
a second bottle containing an aqueous solution containing an aldehyde and preferably a phosphate buffer (said solution being preferably free or substantially free of the said first compound), the content of the said bottles having to be mixed together for preparing the stabilizing solution.

Further details and characteristics of the invention will appear from the following detailed description of particular embodiments, which are described by way of example only.

In this description, reference is made to the annexed drawings. In these drawings,

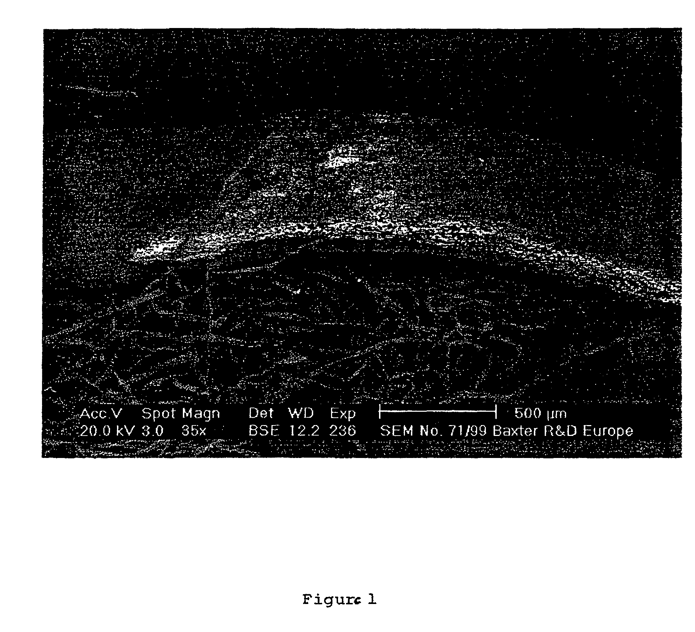

- figure 1 shows a bovine pericardium treated with a glutaraldehyde solution (without tannic acid) after an incubation in a phosphate buffer containing 2.2mM CaCl₂ for 5 weeks,
- figure 2 shows the calcium dosage after incubation for 8 weeks for different treated tissues, and
- figure 3 shows the calcium dosage after incubation for 9 weeks for different treated tissues.

After removal, the pericardium or valve of the pig was rinsed out with an isotonic saline solution. Then, it was cut up into several pieces of tissues.

Glutaraldehyde aqueous solutions were prepared by diluting a concentrated glutaraldehyde solution through the addition of demineralized water thereto, by using a phosphate aqueous buffer solution (sodium phosphate buffer - pH 7.4), and by adding, if need be, gallotannic acid with formula 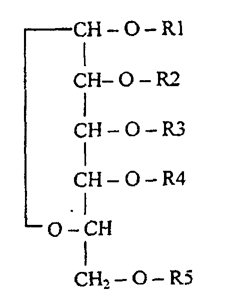 where R1, R2, R3, R4 and R5: the rest of gallic acid -(CO)-(C₆H₅O₃)

### (Reference) tissue 1.

This tissue was treated in an aqueous solution buffered with a phosphate buffer with 0.6% by weight glutaraldehyde (pH of the solution: about 7) for 24 hours at 4°C.

### (Reference) tissue 2.

These tissues were treated in an aqueous solution buffered with a phosphate buffer with 2.0% by weight glutaraldehyde (pH of the solution about 7) for 24 hours at 4°C.

### Tissue 3.

This tissue was treated in an aqueous solution buffered with a phosphate buffer with 0.6% by weight glutaraldehyde (pH of the solution about 7) for 24 hours at 4°C, before treatment in a solution containing 1% by weight of gallotannic acid (in presence of a phosphate buffer, pH of about 7) for 8 hours at 4°C.

### Tissue 4 .

This tissue was treated in an aqueous solution containing 0.6% by weight glutaraldehyde and 1.0% by weight gallotannic acid (in presence of a phosphate buffer, pH of the solution about 7) for 24 hours at 4°C.

### Tissue 5.

This tissue was treated in an aqueous solution containing 2% by weight glutaraldehyde and 1.0% by weight gallotannic acid (in presence of a phosphate buffer, pH of the solution about 7) for 24 hours at 4°C.

### Tissue 6.

This tissue was treated in an aqueous solution containing 1.0% by weight gallotannic acid (in presence of a phosphate buffer, pH of the solution about 7) for 24 hours at 4°C.

After treatment, tissues 1 to 6 were rinsed out with a NaCl solution in sterile physiological demineralized water (physiological saline solution). Tissues were further sterilized by immersion in a 0.6% glutaraldehyde buffer solution for 48 hours at 37°C.

### Determination of in vitro calcification of treated and sterilized tissues.

After treatment, these tissues were placed in Petri dishes containing a solution with 135 millimole of NaCl per liter, 2.2 millimole of CaCl₂2H₂O per liter, 1.2 millimole of KH₂PO₄ per liter, and 0 . 05 millimole of 3-(N-morpholino-propane) sulfonic acid, this solution being buffered at pH 7.4 by means of a phosphate buffer and being sterilized by laminar flow filtration.

Petri dishes are placed in an incubator at 37°C, in a CO₂ atmosphere for 35 days, the solutions in the dishes being refreshed every 7 days under sterile conditions.

The calcification rate of tissues after incubation in the Petri dishes was first determined by examination by a scanning electron microscope provided with an x-ray detector, and by flame emission spectrophotometry through atom absorption.

In order to allow an X-ray examination by scanning electron microscopy, parts of the treated tissues were fastened to an aluminum support, covered by carbon particles of less than 10 nanometers in argon for 60 seconds, and then analyzed by a Philips XL 20 scanning microscope, provided with a ray detector EDAX. This analysis allows to identify and quantify the presence of elements such as calcium and phosphor at the surface of the tissue under examination.

For the spectrophotometric examination, parts of the treated tissues were washed with a sterile saline solution and dried at 90°C in a dessicator. After digestion in a 70% nitric acid solution (3 parts by weight), the calcium content was further determined by flame spectrophotometry (Perkin Elmer device, Zeeman 5100 type).

The preservation of the tissues according to the invention was analyzed by a transmission electron microscope. In order to allow transmission electron microscopic examination, parts of the treated tissues are dehydrated to obtain resin blocks. These blocks are further cut up into extra-thin slices, which are collected in a copper dish, where they are treated with a saturated solution of uranyl alcohol acetate and of lead citrate. Then, the slices were examined by a scanning and transmission electron microscope (Philips EM 300 G).

These examinations showed that the tissues 3 to 6 according to the invention had a lower calcification than reference tissues 1, 2 and that the ultra structure of the tissues 3, 4, 5 and 6 according to the invention was better maintained. Tissues 3, 4, 5, 6 had the lowest calcification and a structure substantially corresponding to the ultra structure of the fresh non-treated tissue.

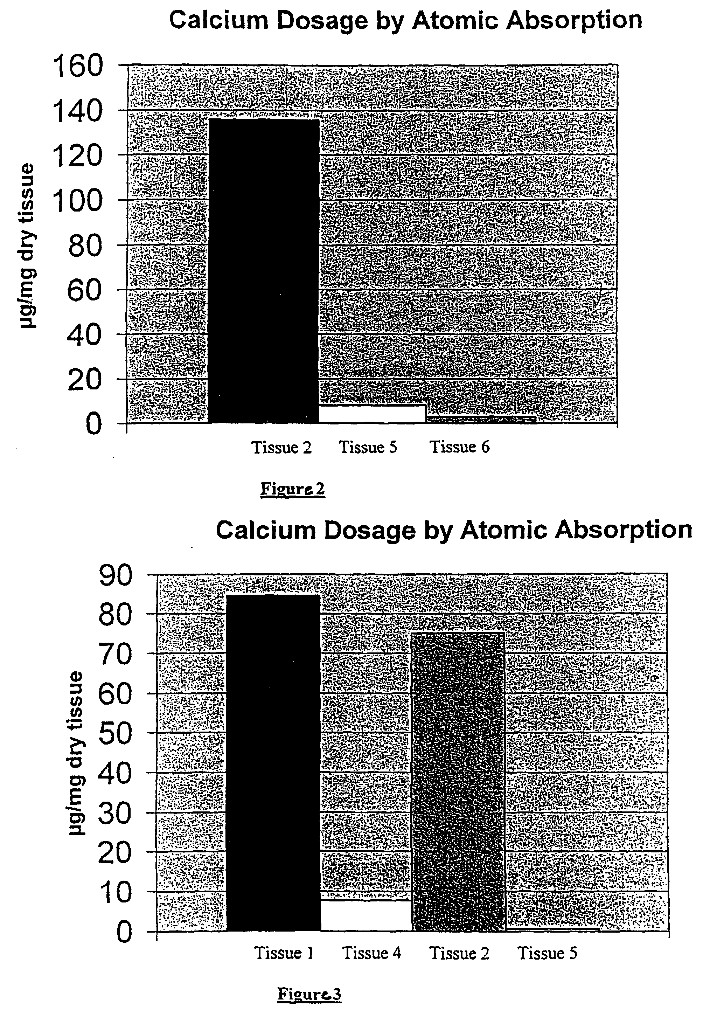

Figure 2 is a graph showing the calcium dosage measured after an incubation of 8 weeks for the tissues 2, 5 and 6. As it can be seen from said graph the calcification of the tissues 5 and 6 was well below 10µg/mg of dry tissue, i.e. more than about 15 times lower than the calcification of the tissue 2.

Figure 3 is a graph showing the calcium dosage measured after an incubation of 9 weeks for the tissues 1, 4, 2 and 5. As it can be seen from said graph the calcification of the tissues 3 and 5 was well below 10µg/mg of dry tissue, i.e. more than about 10 times less than the calcification of the tissue 1 or 2.

It is worthwhile to note that the calcification rate of a tissue treated only by means of glutaraldehyde can vary of more than about 50µg/mg dry tissue for a period of 8 weeks (variation from about 80 to about 140 µg/mg dry tissue), while for tissues according to the invention the calcification rate of a tissue is in any case less than 10µg/mg of dry tissue for a period of 8 weeks.

### Determination of in vivo calcification of treated and sterilized tissues.

In vivo determination was carried out by using rats weighing 200 grams. Each animal received in its dorsal part (cranial part and caudal part), in four distinct subcutaneous pockets two pieces of tissue 6, and two pieces of tissue 1 as reference. After placing the tissue in a pocket, the pocket was closed by means of a suture material Dermalon 2.0, by a needle CE-6 (sold by Cyanamide Bénélux).

The rats were fed with laboratory rat food (Purina Meals Inc.). 21 days after tissue implantation, the rats were killed by a lethal dose of thiopental (300 mg/kg) before getting the samples of implanted tissues from the rats.

As for the in vitro tests, the samples of implanted tissues were submitted to visual examination, to a transmission and scanning electron microscope examination, and to a flame spectrophotometric examination by atom absorption.

This examination showed that tissues 3, 4, 5 and 6 had a lower calcification than the reference tissues 1 and 2.

Parts of tissue 3 and parts of tissue 4 were submitted, before being sterilized with formaldehyde, to halogen lamp irradiation. In this treatment step, the parts of tissues 2 and 3 were immersed in sterile water having a pH of 7.4 and a temperature of about 20°C. The irradiation lasted 48 hours and was performed with an intensity of about 400-600 lumen/hour.

After irradiation, said parts of tissue were rinsed out with sterile injectable water and exposed to gamma irradiation.

Preparations useful for the invention will be described hereafter:

### Example of liquid oral preparation.

200 mg of gallotannic acid with formula 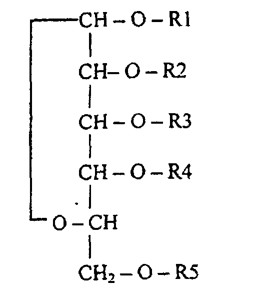 where R1, R2, R3, R4 and R5: the rest of gallic acid -(CO)-(C₆H₅O₃), were dissolved in 100 ml of water containing 1 gram of taste-masking aspartame.

### Examples of solid oral form.

### Example 1

The following ingredients were used to make microgranules:
Gallotannic acid (same formula as oral form): 50 g
Sucroester WE 15 (Gattefosse): 100 g
Avicel PH 101 (FMC microcrystalline cellulose): 100 g
Polyvinyl pyrrolidone K 30: 10 g

These ingredients were put in powder form, into a planetary mixer and granulated by addition of 100 g of distilled water- The plastic mass so obtained was extruded through the cylindrical die, with a diameter of 1 mm, of an extruder (Alexanderwerk). The cylinders so obtained were further transformed into spheres by spheronization in a device of the Maumerizer type. After being dried for 24 hours in a ventilated room, at 50°C, the fraction of microgranules having a diameter of 0.7 to 1.4 mm was separated by screening.

Microgranules (0.7 mm - 1.4 mm) were coated with a porous membrane by spraying thereon, in a fluid bed (Aeromatic, Strea type) a dispersion, composed of:
Talc (lubricant): 5 parts by weight
Polyvinyl pyrrolidone (plasticizer): 0.75 parts by weight
Tween 20 (wetting agent): 0.05 parts by weight
Hydroxypropylmethyl cellulose phatalate HP 55 F (enteric chemical): 7 parts by weight
Eudragit E30D (insoluble polymer): 41 parts by weight
Distilled water: 46 parts by weight
This dispersion was sprayed on the microgranules in the amount of 1 part by weight of dispersion per 2 parts by weight of microgranules. Then, the coated microgranules were dried in an oven at 50°C for 24 hours.

Microganules so obtained, possibly mixed with sucrose granules, were inserted in gelatin capsules. So, capsules containing 200 mg of gallotannic acid (each capsule containing 1.5 g of coated microgranules and 1.5 g of sucrose granules) and capsules containing 400 mg of gallotannic acid (each capsule containing 3 g of coated microgranules) were prepared.

Thanks to their particular porous membrane, the microgranules had prolonged release or liberation properties.

### Example 2

Example 1 has been repeated, except that gallotannic acid of formula 200 mg of gallotannic acid with formula 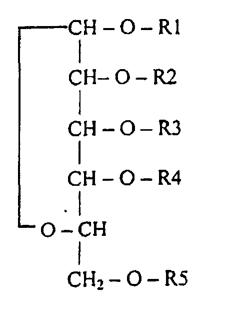 where R1, R2, R3, R4 and R5: the rest of digallic acid, namely - (CO)-(C₆H₄O₂)-O-(CO)-(C₆H₅O₃).

### Example 3

Example 1 has been repeated, except that a mixture of gallotannic acids was used, said mixture containing 50% by weight of the gallotannic acid of example 1 and 50% by weight of the gallotannic acid of example 2.

### Example 4

Example 1 has been repeated, except that vascalagin was used as tannic acid.

### Example 5

Example 1 has been repeated, except that vescalin was used as tannic acid.

### Example of a stabilizing solution

The solution was prepared by mixing the content of a first vial or bottle with the content of a second vial or bottle, the first vial containing 1 g of gallotannic acid of formula 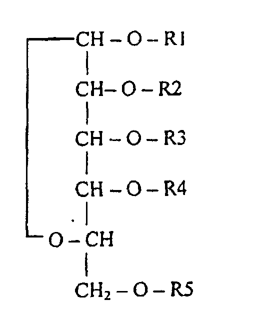 where R1, R2, R3, R4 and R5: the rest of digallic acid, namely - (CO)-(C₆H₄O₂)-O-(CO)-(C₆H₅O₃),
while the second vial contains 100ml of an aqueous solution (prepared by using sterile pyrogen free water) containing 1% by weight glutaraldehyde and a sufficient amount of phosphate buffer (sodium phosphate buffer) so as to obtain after mixing the content of the two vials a solution having a pH of about 7.

The mixing of the content of the two vials can be effected by adding the content of the first vial into the second vial. However, preferably the content of the second vial is introduced in the first vial for rehydration of the gallotanic acid, said hydration step being advantageously carried out at a temperature of 20-50°C, preferably at about 37°C. The complete hydration of the gallotannic acid was obtained in the present case after 15 minutes at 37°C. Preferably, the content of the two vials is shaked.

## Claims

1. A cardiac valve which has a biological or biocompatible support associated to at least one compound having at least one ring of 6 carbon atoms with at least two hydroxyl groups thereon.

2. A cardiac valve as claimed in claim 1, wherein the at least one compound having at least one ring of 6 carbon atoms with at least two hydroxyl groups thereon is present at least at the surface of the biological or biocompatible support.

3. A cardiac valve as claimed in claim 1 or claim 2, wherein the biological or biocompatible support is associated to at least one compound having at least one ring of 6 carbon atoms with at least three hydroxyl groups thereon.

4. A cardiac valve as claimed in claim 1, wherein it is at least partially made from a polymer or copolymer compound or an at least partly cross-linked, biocompatible compound associated to at least one compound having at least one ring of 6 carbon atoms with at least two hydroxyl groups thereon.

5. A cardiac valve as claimed in claim 4, wherein the biological or biocompatible support comprises, at least at its surface, one or more compounds having at least one ring of 6 carbon atoms with at least two hydroxyl groups thereon which are associated to the polymer or copolymer compound or to the at least partly cross-linked, biocompatible compound.

6. A cardiac valve as claimed in claim 4 or claim 5, wherein the polymer or copolymer compound or the at least partly cross-linked, biocompatible compound is associated to at least one compound having at least one ring of 6 carbon atoms with at least three hydroxyl groups thereon.

7. A cardiac valve as claimed in claim 1 or claim 2, wherein it has the form of a biological tissue stabilised at least partially by a polymer or copolymer compound or by an at least partly cross-linked, biocompatible compound associated to at least one compound having at least one ring of 6 carbon atoms with at least two hydroxyl groups thereon.

8. A cardiac valve as claimed in claim 7, wherein the polymer or copolymer compound or the at least partly cross-linked, biocompatible compound is associated to at least one compound having at least one ring of 6 carbon atoms with at least three hydroxyl groups thereon.

9. A cardiac valve as claimed in claim 7, wherein the biological tissue is stabilized at least partially by an aldehyde with the aldehyde at the surface of the tissue or in the proximity thereof being at least partially associated to a compound having at least one ring of 6 carbon atoms with at least two hydroxyl groups thereon.

10. A cardiac valve as claimed in claim 9, wherein the aldehyde at the surface of the tissue or in the proximity thereof is at least partially associated to a compound having at least one ring of 6 carbon atoms with at least three hydroxyl groups thereon.

11. A cardiac valve as claimed in any one of claims 1 to 10, wherein the compound having at least one ring of 6 carbon atoms with at least two hydroxyl groups thereon is selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid and mixtures thereof.

12. A cardiac valve as claimed in claim 11, wherein the compound having at least one ring of 6 carbon atoms with at least two hydroxyl groups thereon is selected from the group consisting of tannic acids, salts of tannic acids, esters of tannic acids, hydrolysis products of said salts and esters, and mixtures thereof.

13. A cardiac valve as claimed in claim 11, wherein the compound having at least one ring of 6 carbon atoms with at least two hydroxyl groups thereon is selected from the group consisting of tannic acids with the formula: 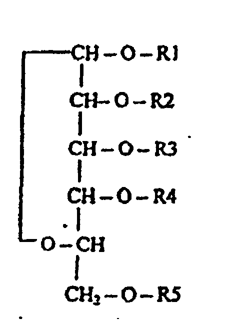 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

14. A cardiac valve as claimed in any one of claims 2, 5, 7 and 9, wherein it comprises at its surface a layer containing at least one compound selected from the group consisting of tannic acids with the formula: 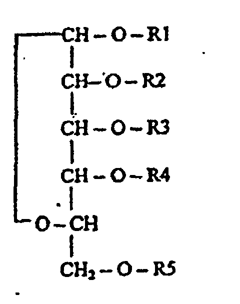 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

15. A cardiac valve as claimed in any one of claims 1 to 10 which takes the form of a body having at its surface and inside the body one or more compounds selected from the group consisting of tannic acids with the formula: 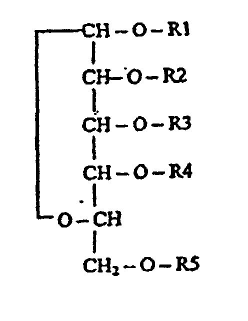 where each of R¹, R², R³, R⁴ and R⁵ are radicals obtained after removal of the hydroxyl group from the carboxyl function of gallic acid or digallic acid, salts and esters of these tannic acids; quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

16. Use of a biocompatible support which is associated to at least one compound having at least one ring of 6 carbon atoms with at least two hydroxyl groups thereon in the manufacture of an animal or human implant.

17. The use as claimed in claim 16, wherein the biocompatible support is associated to at least one compound having at least one ring of 6 carbon atoms with at least three hydroxyl groups thereon.

18. The use as claimed in claim 16, wherein the implant has the form of a biological tissue stabilised at least partially by a polymer or copolymer compound or by an at least partly cross-linked, biocompatible compound associated to at least one compound having at least one ring of 6 carbon atoms with at least two hydroxyl groups thereon.

19. The use as claimed in claim 18, wherein the polymer or copolymer compound or the at least partly cross-linked, biocompatible compound is associated to at least one compound having at least one ring of 6 carbon atoms with at least three hydroxyl groups thereon.

20. The use as claimed in claim 18, wherein the biological tissue is stabilised at least partially by an aldehyde with the aldehyde at the surface of the tissue or in the proximity thereof being at least partially associated to a compound having at least one ring of 6 carbon atoms with at least two hydroxyl groups thereon.

21. The use as claimed in claim 20, wherein the aldehyde at the surface of the tissue or in the proximity thereof is at least partially associated to a compound having at least one ring of 6 carbon atoms with at least three hydroxyl groups thereon.

22. The use as claimed in claim 16 or claim 17, wherein the support comprises a polymer or copolymer compound or an at least partly cross-linked, biocompatible compound.

23. The use as claimed in any one of claims 16 to 22, wherein the implant is a cardiac valve.

24. The use as claimed in any one of claims 16 to 23, wherein the at least one compound having at least one ring of 6 carbon atoms with at least two hydroxyl groups thereon is present at least at the surface of the implant.

25. The use as claimed in any one of claims 16 to 24, wherein the compound having at least one ring of 6 carbon atoms with at least two hydroxyl groups thereon is selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid and mixtures thereof.

26. The use as claimed in claim 25, wherein the compound having at least one ring of 6 carbon atoms with at least two hydroxyl groups thereon is selected from the group consisting of hydrolysable tannic acids, salts of hydrolysable tannic acids, esters of hydrolysable tannic acids, hydrolysis products of said salts and esters, vescalin, vascalagin, hydrolysis products of vescalin or vascalagin, esters and salts of vescalin and vascalagin, condensation product of an aldehyde with tannins or tannic acid, and mixtures thereof.

27. The use as claimed in claim 25, wherein the compound having at least one ring of 6 carbon atoms with at least two hydroxyl groups thereon is selected from the group consisting of tannic acids with the formula: 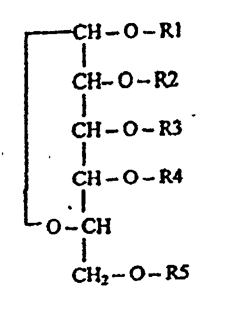 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

28. The use as claimed in any one of claims 16 to 24, wherein the implant comprises at its surface a layer containing at least one compound selected from the group consisting of tannic acids with the formula: 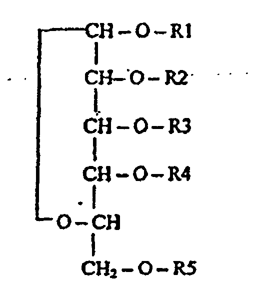 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

29. The use as claimed in any one of claims 16 to 24, wherein the implant takes the form of a body having at its surface and inside the body one or more compounds selected from the group consisting of tannic acids with the formula: 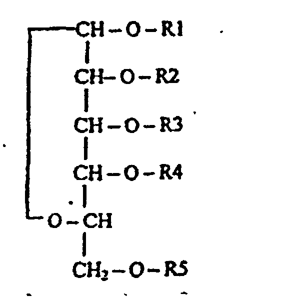 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof

30. A method for preparing an animal or human implant having a support, said method comprising the steps of:
treating the implant with a solution containing a compound having at least one ring of 6 carbon atoms with at least two hydroxyl groups thereon; and
following said treatment sterilising said implant and/or treating said implant aseptically.

31. A method as claimed in claim 30, wherein the implant is treated with a solution containing a compound having at least one ring of 6 carbon atoms with at least three hydroxyl groups thereon.

32. A method as claimed in claim 30, wherein the implant is at least partially prepared from a polymer or copolymer compound or from a cross-linked, biocompatible compound which have been at least partially treated with a compound having at least one ring of 6 carbon atoms with at least two hydroxyl groups thereon.

33. A method as claimed in claim 32, wherein the polymer or copolymer compound and the cross-linked, biocompatible compound have been at least partially treated with a compound having at least one ring of 6 carbon atoms with at least three hydroxyl groups thereon.

34. A method as claimed in claim 32 or claim 33, wherein the support is associated to at least the polymer or copolymer compound or to the partly cross-linked, biocompatible compound.

35. A method as claimed in any one of claims 30 to 34, wherein a biological tissue that has been at least partially stabilised by an aldehyde is used for the implant.

36. A method as claimed in any one of claims 30 to 35, wherein the compound having at least one ring of 6 carbon atoms with at least two hydroxyl groups thereon is selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin; esters and salts of vescalin and vascalagin, condensation product of an aldehyde with said tannins or tannic acids and mixtures thereof.

37. A method as claimed in claim 36, wherein the compound having at least one ring of 6 carbon atoms with at least two hydroxyl groups thereon is selected from the group consisting of tannic acids, salts of tannic acids, esters of tannic acids, hydrolysis products of said salts and esters, and mixtures thereof.

38. A method as claimed in any one of claims 30 to 35, wherein the compound having at least one ring of 6 carbon atoms with at least two hydroxyl groups thereon is selected from the group consisting of tannic acids with the formula: 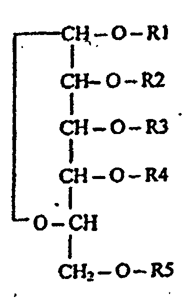 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

39. A method as claimed in any one of claims 30 to 39, wherein the solution that is used for treating the implant has a pH of from 3 to 9.

40. A method as claimed in any one of claims 30 to 39, wherein the solution that is used for treating the implant has a pH of from 5.5 to 7.5.

41. Use of at least one compound selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid and mixtures thereof in the manufacture of a pharmaceutical composition containing an effective amount of said at least one compound for treating or preventing calcification in a blood circuit.

42. The use as claimed in claim 41, wherein the at least one compound is selected from the group consisting of tannic acids with the formula: 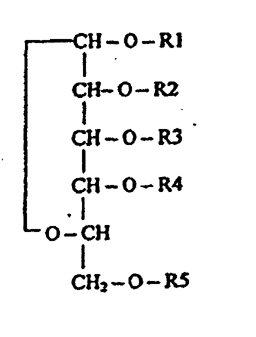 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

43. The use as claimed in claim 42, wherein the pharmaceutical composition is used for treating or preventing calcification of a cardiac valve and/or an implant in contact with blood.

44. The use as claimed in claim 42, wherein the at least one compound is selected from the group consisting of tannic acids with the formula: 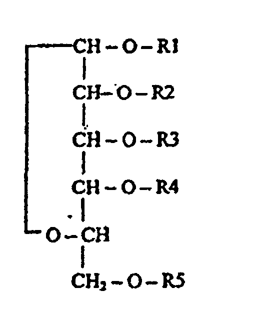 where each of R¹, R², R³, R⁴ and R⁵ are radicals obtained after removal of the hydroxyl group from the carboxyl function of gallic acid or digallic acid, salts and esters of these tannic acids, hydrolysis products of the salts and esters, vescalin, vascalagin, hydrolysis products of vescalin or vascalagin, esters and salts of vescalin and vascalagin, condensation product of an aldehyde with tannins or tannic acid and mixtures thereof.

45. The use as claimed in any one of claims 41 to 44, wherein the composition has the form of a prolonged release composition.

46. An implantable support for contact with a biological medium, said support being associated to at least one compound having at least one ring of 6 carbon atoms with at least two hydroxyl groups thereon.

47. An implantable support as claimed in claim 46, wherein the biological medium is a human or an animal.

48. An implantable support as claimed in claim 46 or claim 47, wherein the at least one compound having at least one ring of 6 carbon atoms with at least two hydroxyl groups is present at at least one of the surfaces of the support.

49. An implantable support as claimed in claim 48, wherein the at least one compound having at least one ring of 6 carbon atoms with at least two hydroxyl groups thereon is associated to a polymer or copolymer compound or to an at least partly cross-linked, biocompatible compound.

50. An implantable support as claimed in any one of claims 46 to 48 comprising a polymer or copolymer compound and/or an at least partly cross-linked, biocompatible compound.

51. An implantable support as claimed in any one of claims 46 to 50, wherein the support is associated to at least one compound having at least one ring of 6 carbon atoms with at least three hydroxyl groups thereon.

52. An implantable support as claimed in any one of claims 46 to 48 which is a biological implantable support.

53. An implantable support as claimed in any one of claims 46 to 48, wherein the support has the form of a biological tissue stabilised at least partially by a polymer or copolymer compound or by an at least partly cross-linked, biocompatible compound associated to at least one compound having at least one ring of 6 carbon atoms with at least two hydroxyl groups thereon.

54. An implantable support as claimed in claim 53, wherein the polymer or copolymer compound or the at least partly cross-linked, biocompatible compound is associated to at least one compound having at least one ring of 6 carbon atoms with at least three hydroxyl groups thereon.

55. An implantable support as claimed in claim 53 or claim 54, wherein the biological tissue is stabilized at least partially by an aldehyde with at least the aldehyde which is at the surface of the tissue or in the proximity thereof being at least partially associated to a compound having at least one ring of 6 carbon atoms with at least two hydroxyl groups thereon.

56. An implantable support as claimed in claim 55, wherein the aldehyde at the surface of the tissue or in the proximity thereof is at least partially associated to a compound having at least one ring of 6 carbon atoms with at least three hydroxyl groups thereon.

57. An implantable support as claimed in any one of claims 46 to 56, wherein the compound having at least one ring of 6 carbon atoms with at least two hydroxyl groups thereon is selected from the group consisting of tannins, tannic acids, salts of tannic acids, esters of tannic acids, hydrolysis products of salts and esters of tannic acids and tannins, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, shikimic acid, dehydroshikimic acid, salts and esters of shikimic acid and of dehydroshikimic acid, vescalin, vascalagin, hydrolysis products of vescalin or vascalagin, esters and salts of vescalin and vascalagin, condensation product of an aldehyde with said tannins or tannic acid, and mixtures thereof.

58. The support of claim 57, wherein the compound having at least one ring of 6 carbon atoms with at least two hydroxyl groups thereon is selected from the group consisting of hydrolysable tannic acids, salts of hydrolysable tannic acids, esters of hydrolysable tannic acids, hydrolysis products of said salts and esters, vescalin, vascalagin, hydrolysis products of vescalin or vascalagin, esters and salts of vescalin and vascalagin, condensation product of an aldehyde with said tannins or tannic acid, and mixtures thereof.

59. An implantable support as claimed in claim 57, wherein the compound having at least one ring of 6 carbon atoms with at least two hydroxyl groups thereon is selected from the group consisting of tannic acids with the formula: 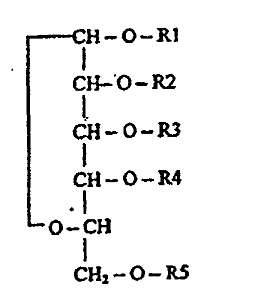 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

60. An implantable support as claimed in any one of claims 46 to 56, wherein it comprises at its surface a layer containing at least one compound selected from the group consisting of tannic acids with the formula: 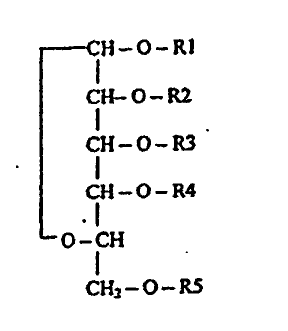 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

61. An implantable support as claimed in any one of claims 46 to 56 which takes the form of a body having at its surface and inside the body one or more compounds selected from the group consisting of tannic acids with the formula: 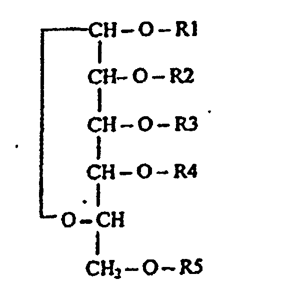 where each of R¹, R², R³, R⁴ and R⁵ are radicals obtained after removal of the hydroxyl group from the carboxyl function of gallic acid or digallic acid, salts and esters of these tannic acids; quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, condensation product of an aldehyde with tannins or tannic acid and mixtures thereof.

62. Use of an aqueous composition for stabilizing an implantable support selected from the group consisting of supports intended to be in contact with a biological medium, biological supports, supports of animal tissue and supports of human tissue, said composition comprising an aldehyde in mixture with at least one compound selected from the group consisting of tannic acids with the formula: 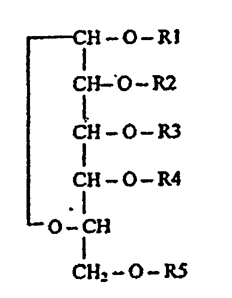 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin, and the condensation product of an aldehyde with tannins or tannic acid.

63. The use as claimed in claim 62, wherein the aqueous composition comprises at least one compound selected from the group consisting of tannic acids with the formula: 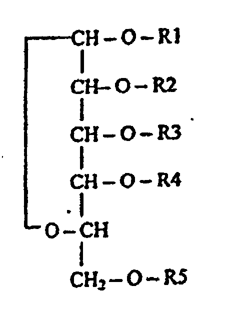 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, esters and salts of vescalin and vascalagin in mixture with a condensation product of an aldehyde with tannins or tannic acid.

64. The use as claimed in claim 62 or claim 63, wherein the pH of the composition is from 3 to 9.

65. The use as claimed in claim 62 or claim 63, wherein the pH of the composition is from 5.5 and 7.5.

66. The use as claimed in claim 62 or claim 63, wherein the pH of the composition is about 7.

67. The use of any one of claims 62 to 66, wherein the aqueous composition contains up to 10% by weight of a first component consisting of at least one compound selected from the group consisting of tannic acids with the formula: 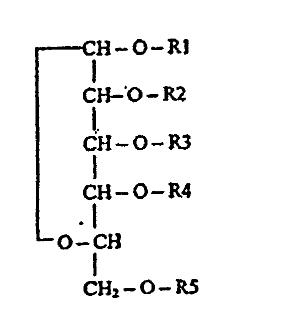 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, and esters and salts of vescalin and vascalagin and up to 10 % by weight of a second component consisting of an aldehyde, a condensation product of an aldehyde with a tannin or a tannic acid or a mixture thereof.

68. The use as claimed in claim 67, wherein the aqueous composition contains less than 5 % by weight of the first component.

69. The use as claimed in claim 68, wherein the aqueous composition contains less than 2.5 % by weight of the first component.

70. The use as claimed in any one of claims 67 to 69, wherein the aqueous composition contains less than 5 % by weight of the second component.

71. The use as claimed in claim 70, wherein the aqueous composition contains less than 2.5 % by weight of the second component.

72. The use as claimed in any one of claims 67 to 71, wherein the weight ratio of the first component to the aldehyde is between 1:10 and 10:1.

73. The use as claimed in claim 72, wherein the weight ratio of the first component to the aldehyde is between 1:5 and 5:1.

74. The use as claimed in any one of claims 62 to 73, wherein the composition contains a phosphate buffer.

75. The use of a kit for preparing a composition for stabilising an implantable support as in any one of claims 62 to 74, said kit comprising first and second bottles having contents to be mixed together for preparing the stabilising composition, wherein:
the first bottle contains, as a powder or in an aqueous solution, at least one compound selected from the group consisting of tannic acids with the formula: 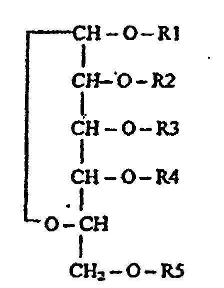 where each of R¹, R², R³, R⁴ and R⁵ are radicals obtained after removal of the hydroxyl group from the carboxyl function of gallic acid or digallic acid, salts and esters of these tannic acids, quinic acid, dehydroquinic acid, esters and salts of quinic acid and of dehydroquinic acid, hydrolysis products of esters and salts of quinic acid and of dehydroquinic acid, gallic acid, digallic acid, esters and salts of gallic acid and of digallic acid, hydrolysis products of esters and salts of gallic acid and of digallic acid, vescalin, vascalagin, hydrolysis products of vescalin or vascalagin, and esters and salts of vescalin and vascalagin, and
the second bottle contains an aqueous solution containing an aldehyde.

76. The use as claimed in claim 75, wherein the second bottle additionally comprises a phosphate buffer.

## Patentansprüche

1. Herzklappe, die einen biologische oder biokompatiblen Träger hat, der mindestens einer Verbindung zugeordnet ist, die mindestens einen Ring von 6 Kohlenstoffatomen mit mindestes zwei Hydroxylgruppen daran hat.

2. Herzklappe nach Anspruch 1, wobei die mindestens eine Verbindung, die mindestens einen Ring von 6 Kohlenstoffatomen mit mindestens zwei Hydroxylgruppen daran hat, mindestens an der Oberfläche des biologischen oder biokompatiblen Trägers anwesend ist.

3. Herzklappe nach Anspruch 1 oder 2, wobei der biologische oder biokompatible Träger mindestens einer Verbindung zugeordnet ist, die mindestens einen Ring von 6 Kohlenstoffatomen mit mindestens drei Hydroxylgruppen daran hat.

4. Herzklappe nach Anspruch 1, wobei sie mindestens teilweise aus einer Polymer- oder Copolymerverbindung oder einer mindestens teilweise vernetzten, biokompatiblen Verbindung besteht, die mindestens einer Verbindung zugeordnet ist, die mindestens einen Ring von 6 Kohlenstoffatomen mit mindestens zwei Hydroxylgruppen daran hat.

5. Herzklappe nach Anspruch 4, wobei der biologische oder biokompatible Träger mindestens an seiner Oberfläche eine oder mehrere Verbindungen aufweist, die mindestens einen Ring von 6 Kohlenstoffatomen mit mindestens zwei Hydroxylgruppen daran haben und der Polymer- oder Copolymerverbindung oder der mindestens teilweise vernetzten, biokompatiblen Verbindung zugeordnet sind.

6. Herzklappe nach Anspruch 4 oder 5, wobei die Polymer- oder Copolymerverbindung oder die mindestens teilweise vernetzte, biokompatible Verbindung mindestens einer Verbindung zugeordnet ist, die mindestens einen Ring von 6 Kohlenstoffatomen mit mindestens drei Hydroxylgruppe daran hat.

7. Herzklappe nach Anspruch 1 oder 2, wobei sie in Form eines biologischen Gewebes ist, das mindestens teilweise stabilisiert ist durch eine Polymer- oder Copolymerverbindung oder durch eine mindestens teilweise vernetzte, biokompatible Verbindung, die mindestens einer Verbindung zugeordnet ist, die mindestens einen Ring von 6 Kohlenstoffatomen mit mindestens zwei Hydroxylgruppen daran hat.

8. Herzklappe nach Anspruch 7, wobei die Polymer- oder Copolymerverbindung oder die mindestens teilweise vernetzte, biokompatible Verbindung mindestens einer Verbindung zugeordnet ist, die mindestens einen Ring von 6 Kohlenstoffatomen mit mindestens drei Hydroxylgruppen daran hat.

9. Herzklappe nach Anspruch 7, wobei das biologische Gewebe mindestens teilweise durch einen Aldehyd stabilisiert ist, wobei der Aldehyd an der Oberfläche des Gewebes oder in der Nähe davon mindestens teilweise einer Verbindung zugeordnet ist, die mindestens einen Ring von 6 Kohlenstoffatomen mit mindestens zwei Hydroxylgruppen daran hat.

10. Herzklappe nach Anspruch 9, wobei der Aldehyd an der Oberfläche des Gewebes oder in der Nähe davon mindestens teilweise einer Verbindung zugeordnet ist, die mindestens einen Ring von 6 Kohlenstoffatomen mit mindestens drei Hydroxylgruppen daran hat.

11. Herzklappe nach einem der Ansprüche 1 bis 10, wobei die Verbindung, die mindestens einen Ring von 6 Kohlenstoffatomen mit mindestens zwei Hydroxylgruppen daran hat, aus der Gruppe ausgewählt ist, die besteht aus Tanninen, Tanninsäuren, Salzen von Tanninsäuren, Estern von Tanninsäuren, Hydrolyseprodukten von Salzen und Estern von Tanninsäuren und Tanninen, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Shikimisäure, Dehydroshikimisäure, Salzen und Estern von Shikimisäure und von Dehydroshikimisäure, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder der Tanninsäure und Gemischen davon.

12. Herzklappe nach Anspruch 11, wobei die Verbindung, die mindestens einen Ring von 6 Kohlenstoffatomen mit mindestens zwei Hydroxylgruppen daran hat, aus der Gruppe ausgewählt ist, die besteht aus Tanninsäuren, Salzen von Tanninsäuren, Estern von Tanninsäuren, Hydrolyseprodukten der Salze und Ester, und Gemischen davon.

13. Herzklappe nach Anspruch 11, wobei die Verbindung, die mindestens einen Ring von 6 Kohlenstoffatomen mit mindestens zwei Hydroxylgruppen daran hat, aus der Gruppe ausgewählt ist, die aus Tanninsäuren mit der folgenden Formel besteht: 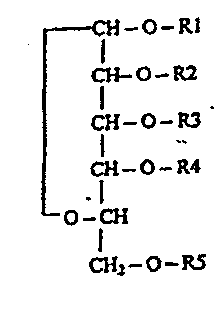 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

14. Herzklappe nach einem der Ansprüche 2, 5, 7 und 9, wobei sie an ihrer Oberfläche eine Schicht aufweist, die mindestens eine Verbindung enthält, die aus der Gruppe ausgewählt ist, die aus Tanninsäuren mit der folgenden Formel besteht: 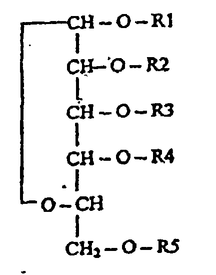 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

15. Herzklappe nach einem der Ansprüche 1 bis 10, die in Form eines Körpers ist, der an seiner Oberfläche und im Inneren des Körpers eine oder mehrere Verbindungen hat, die aus der Gruppe ausgewählt sind, die aus Tanninsäuren mit der folgenden Formel bestehen: 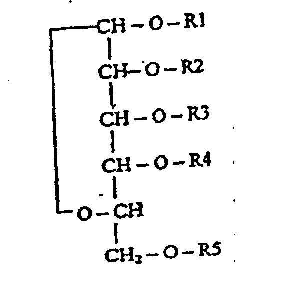 Wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren; Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds von Tanninen oder Tanninsäure und Gemischen davon.

16. Verwendung eines biokompatiblen Trägers, der mindestes einer Verbindung zugeordnet ist, die mindestens einen Ring von 6 Kohlenstoffatomen mit mindestens zwei Hydroxylgruppen daran hat, bei der Herstellung eines Tieroder Human-Implantats.

17. Verwendung nach Anspruch 16, wobei der biokompatible Träger mindestens einer Verbindung zugeordnet ist, der mindestens einen Ring von 6 Kohlenstoffatomen mit mindestens drei Hydroxylgruppen daran hat.

18. Verwendung nach Anspruch 16, wobei das Implantat in Form eines biologischen Gewebes ist, das mindestens teilweise stabilisiert ist durch eine Polymer- oder Copolymerverbindung oder durch eine mindestens teilweise vernetzte, biokompatible Verbindung, die mindestens einer Verbindung zugeordnet ist, die mindestens einen Ring von 6 Kohlenstoffatomen mit mindestens zwei Hydroxylgruppen daran hat.

19. Verwendung nach Anspruch 18, wobei die Polymer- oder Copolymerverbindung oder die mindestens teilweise vernetzte, biokompatible Verbindung mindestens einer Verbindung zugeordnet ist, die mindestens einen Ring von 6 Kohlenstoffatomen mit mindestens drei Hydroxylgruppen daran hat.

20. Verwendung nach Anspruch 18, wobei das biologische Gewebe mindestens teilweise durch einen Aldehyd stabilisiert ist, wobei der Aldehyd an der Oberfläche des Gewebes oder in der Nähe davon mindestens teilweise einer Verbindung zugeordnet ist, die mindestens einen Ring von 6 Kohlenstoffatomen mit mindestens zwei Hydroxylgruppen daran hat.

21. Verwendung nach Anspruch 20, wobei der Aldehyd an der Oberfläche des Gewebes oder in der Nähe davon mindestens teilweise einer Verbindung zugeordnet ist, die mindestens einen Ring von 6 Kohlenstoffatomen mit mindestens drei Hydroxylgruppen daran hat.

22. Verwendung nach Anspruch 16 oder 17, wobei der Träger eine Polymeroder Copolymerverbindung oder mindestens eine teilweise vernetzte, biokompatible Verbindung aufweist.

23. Verwendung nach einem der Ansprüche 16 bis 22, wobei das Implantat eine Herzklappe ist.

24. Verwendung nach einem der Ansprüche 16 bis 23, wobei die mindestens eine Verbindung, die mindestens einen Ring von 6 Kohlenstoffatomen mit mindestens zwei Hydroxylgruppen daran hat, mindestens an der Oberfläche des Implantats anwesend ist.

25. Verwendung nach einem der Ansprüche 16 bis 24, wobei die Verbindung, die mindestens einen Ring von 6 Kohlenstoffatomen mit mindestens zwei Hydroxylgruppen daran hat, aus der Gruppe ausgewählt ist, die besteht aus Tanninen, Tanninsäuren, Salzen von Tanninsäuren, Estern von Tanninsäuren, Hydrolyseprodukten von Salzen und Estern von Tanninsäuren und Tanninen, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Shikimisäure, Dehydroshikimisäure, Salzen und Estern von Shikimisäure und von Dehydroshikimisäure, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder der Tanninsäure und Gemischen davon.

26. Verwendung nach Anspruch 25, wobei die Verbindung, die mindestens einen Ring von 6 Kohlenstoffatomen mit mindestens zwei Hydroxylgruppen daran hat, aus der Gruppe ausgewählt ist, die besteht aus hydrolisierbaren Tanninsäuren, Salzen von hydrolisierbaren Tanninsäuren, Estern von hydrolisierbaren Tanninsäuren, Hydrolyseprodukten der Salze und Ester, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure, und Gemischen davon.

27. Verwendung nach Anspruch 25, wobei die Verbindung, die mindestens einen Ring von 6 Kohlenstoffatomen mit mindestens zwei Hydroxylgruppen daran hat, aus der Gruppe ausgewählt ist, die aus Tanninsäuren mit der folgenden Formel besteht: 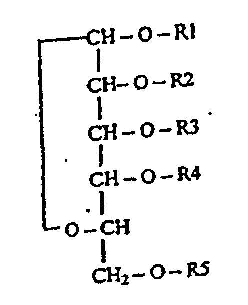 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

28. Verwendung nach einem der Ansprüche 16 bis 24, wobei das Implantat an seiner Oberfläche eine Schicht aufweist, die mindestens eine Verbindung enthält, die aus der Gruppe ausgewählt ist, die aus Tanninsäuren mit der folgenden Formel besteht: 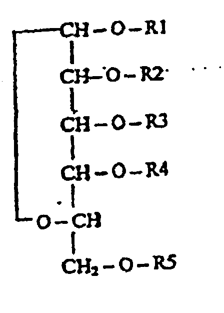 wobei R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

29. Verwendung nach einem der Ansprüche 16 bis 24, wobei das Implantat in Form eines Körpers ist, der an seiner Oberfläche und im Inneren des Körpers eine oder mehrere Verbindungen hat, die aus der Gruppe ausgewählt sind, die aus Tanninsäuren mit der folgenden Formel bestehen: 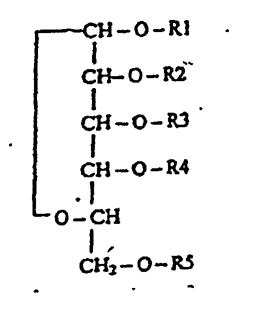 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

30. Verfahren zum Herstellen eines Tier- oder Human-Implantats, das einen Träger hat, wobei das Verfahren die folgenden Schritte aufweist:
Behandeln des Implantats mit einer Lösung, die eine Verbindung enthält, die mindestens einen Ring von 6 Kohlenstoffatomen mit mindestens zwei Hydroxylgruppen daran hat; und
im Anschluß an diese Behandlung Sterilisieren des Implantats und/oder aseptisches Behandeln des Implantats.

31. Verfahren nach Anspruch 30, wobei das Implantat mit einer Lösung behandelt wird, die eine Verbindung enthält, die mindestens einen Ring von 6 Kohlenstoffatomen mit mindestens drei Hydroxylgruppen daran hat.

32. Verfahren nach Anspruch 30, wobei das Implantat mindestens teilweise hergestellt wird aus einer Polymer- oder Copolymerverbindung oder aus einer vernetzten, biokompatiblen Verbindung, die mindestens teilweise mit einer Verbindung behandelt worden sind, die mindestens einen Ring von 6 Kohlenstoffatomen mit mindestens zwei Hydroxylgruppen daran hat.

33. Verfahren nach Anspruch 32, wobei die Polymer- oder Copolymerverbindung und die vernetzte, biokompatible Verbindung mindestens teilweise mit einer Verbindung behandelt worden sind, die mindestens einen Ring von 6 Kohlenstoffatomen mit mindestens drei Hydroxylgruppen daran hat.

34. Verfahren nach Anspruch 32 oder 33, wobei der Träger mindestens der Polymer- oder Copolymerverbindung oder der teilweise vernetzten, biokompatiblen Verbindung zugeordnet wird.

35. Verfahren nach einem der Ansprüche 30 bis 34, wobei ein biologisches Gewebe, das mindestens teilweise durch einen Aldehyd stabilisiert worden ist, für das Implantat verwendet wird.

36. Verfahren nach einem der Ansprüche 30 bis 35, wobei die Verbindung, die mindestens einen Ring von 6 Kohlenstoffatomen mit mindestens zwei Hydroxylgruppen daran hat, aus der Gruppe ausgewählt ist, die besteht aus Tanninen, Tanninsäuren, Salzen von Tanninsäuren, Estern von Tanninsäuren, Hydrolyseprodukten von Salzen und Estern von Tanninsäuren und Tanninen, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Shikimisäure, Dehydroshikimisäure, Salzen und Estern von Shikimisäure und von Dehydroshikimisäure, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder Tanninsäuren und Gemischen davon.

37. Verfahren nach Anspruch 36, wobei die Verbindung, die mindestens einen Ring von 6 Kohlenstoffatomen mit mindestens zwei Hydroxylgruppen daran hat, aus der Gruppe ausgewählt ist, die besteht aus Tanninsäuren, Salzen von Tanninsäuren, Estern von Tanninsäuren, Hydrolyseprodukten der Salze und Ester, und Gemischen davon.

38. Verfahren nach einem der Ansprüche 30 bis 35, wobei die Verbindung, die mindestens einen Ring von 6 Kohlenstoffatomen mit mindestens zwei Hydroxylgruppen daran hat, aus der Gruppe ausgewählt ist, die aus Tanninsäuren mit der folgenden Formel besteht: 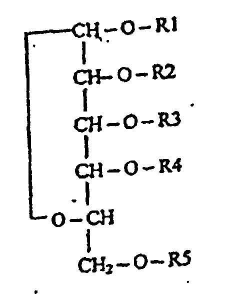 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

39. Verfahren nach einem der Ansprüche 30 bis 39, wobei die Lösung, die zum Behandeln des Implantats verwendet wird, einen pH-Wert von 3 bis 9 hat.

40. Verfahren nach einem der Ansprüche 30 bis 39, wobei die Lösung, die zum Behandeln des Implantats verwendet wird, einen pH-Wert von 5,5 bis 7,5 hat.

41. Verwendung von mindestens einer Verbindung, die aus der Gruppe ausgewählt ist, die besteht aus Tanninen, Tanninsäuren, Salzen von Tanninsäuren, Estern von Tanninsäuren, Hydrolyseprodukten von Salzen und Estern von Tanninsäuren und Tanninen, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Shikimisäure, Dehydroshikimisäure, Salzen und Estern von Shikimisäure und von Dehydroshikimisäure, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder der Tanninsäure und Gemischen davon, bei der Herstellung einer pharmazeutischen Zusammensetzung, die eine wirksame Menge der mindestens einen Verbindung zum Behandeln oder Verhindern von Kalzifizierung in einem Blutkreislauf enthält.

42. Verwendung nach Anspruch 41, wobei die mindestens eine Verbindung aus der Gruppe ausgewählt ist, die aus Tanninsäuren mit der folgenden Formel besteht: 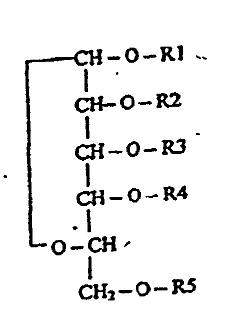 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

43. Verwendung nach Anspruch 42, wobei die pharmazeutische Zusammensetzung zum Behandeln oder Verhindern von Kalzifizierung einer Herzklappe und/oder eines Implantats in Kontakt mit Blut verwendet wird.

44. Verwendung nach Anspruch 42, wobei die mindestens eine Verbindung aus der Gruppe ausgewählt ist, die aus Tanninsäuren mit der folgenden Formel besteht: 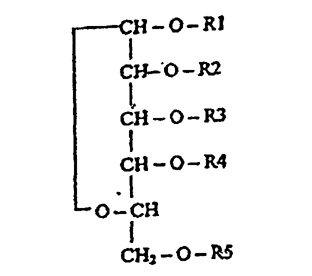 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Hydrolyseprodukten der Salze und Ester, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

45. Verwendung nach einem der Ansprüche 41 bis 44, wobei die Zusammensetzung in Form einer prolonged-release-Zusammensetzung ist.

46. Implantierbarer Träger zum Kontakt mit einem biologischen Medium, wobei der Träger mindestens einer Verbindung zugeordnet ist, die mindestens einen Ring von 6 Kohlenstoffatomen mit mindestens zwei Hydroxylgruppen daran hat.

47. Implantierbarer Träger nach Anspruch 46, wobei das biologische Medium ein Mensch oder ein Tier ist.

48. Implantierbarer Träger nach Anspruch 46 oder 47, wobei die mindestens eine Verbindung, die mindestens einen Ring von 6 Kohlenstoffatomen mit mindestens zwei Hydroxylgruppen hat, an mindestens einer der Oberflächen des Trägers anwesend ist.

49. Implantierbarer Träger nach Anspruch 48, wobei die mindestens eine Verbindung, die mindestens einen Ring von 6 Kohlenstoffatomen mit mindestens zwei Hydroxylgruppen daran hat, einer Polymer- oder Copolymerverbindung oder einer mindestens teilweise vernetzten, biokompatiblen Verbindung zugeordnet ist.

50. Implantierbarer Träger nach einem der Ansprüche 46 bis 48, der eine Polymer- oder Copolymerverbindung und/oder eine mindestens teilweise vernetzte, biokompatible Verbindung aufweist.

51. Implantierbarer Träger nach einem der Ansprüche 46 bis 50, wobei der Träger mindestens einer Verbindung zugeordnet ist, die mindestens einen Ring von 6 Kohlenstoffatomen mit mindestens drei Hydroxylgruppen daran hat.

52. Implantierbarer Träger nach einem der Ansprüche 46 bis 48, der ein biologischer implantierbarer Träger ist.

53. Implantierbarer Träger nach einem der Ansprüche 46 bis 48, wobei der Träger in Form eines biologischen Gewebes ist, das mindestens teilweise stabilisiert ist durch eine Polymer- oder Copolymerverbindung oder durch eine mindestens teilweise vernetzte, biokompatible Verbindung, die mindestens einer Verbindung zugeordnet ist, die mindestens einen Ring von 6 Kohlenstoffatomen mit mindestens zwei Hydroxylgruppen daran hat.

54. Implantierbarer Träger nach Anspruch 53, wobei die Polymer- oder Copolymerverbindung oder die mindestens teilweise vernetzte, biokompatible Verbindung mindestens einer Verbindung zugeordnet ist, die mindestens einen Ring von 6 Kohlenstoffatomen mit mindestens drei Hydroxylgruppen daran hat.

55. Implantierbarer Träger nach Anspruch 53 oder 54, wobei das biologische Gewebe mindestens teilweise durch einen Aldehyd stabilisiert ist, wobei mindestens der Aldehyd, der an der Oberfläche des Gewebes oder in der Nähe davon ist, mindestens teilweise einer Verbindung zugeordnet ist, die mindestens einen Ring von 6 Kohlenstoffatomen mit mindestens zwei Hydroxylgruppen daran hat.

56. Implantierbarer Träger nach Anspruch 55, wobei der Aldehyd an der Oberfläche des Gewebes oder in der Nähe davon mindestens teilweise einer Verbindung zugeordnet ist, die mindestens einen Ring von 6 Kohlenstoffatomen mit mindestens drei Hydroxylgruppen daran hat.

57. Implantierbarer Träger nach einem der Ansprüche 46 bis 56, wobei die Verbindung, die mindestens einen Ring von 6 Kohlenstoffatomen mit mindestens zwei Hydroxylgruppen daran hat, aus der Gruppe ausgewählt ist, die besteht aus Tanninen, Tanninsäuren, Salzen von Tanninsäuren, Estern von Tanninsäuren, Hydrolyseprodukten von Salzen und Estern von Tanninsäuren und Tanninen, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Shikimisäure, Dehydroshikimisäure, Salzen und Estern von Shikimisäure und von Dehydroshikimisäure, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder der Tanninsäure, und Gemischen davon.

58. Träger nach Anspruch 57, wobei die Verbindung, die mindestens einen Ring von 6 Kohlenstoffatomen mit mindestens zwei Hydroxylgruppen daran hat, aus der Gruppe ausgewählt ist, die besteht aus hydrolisierbaren Tanninsäuren, Salzen von hydrolisierbaren Tanninsäuren, Estern von hydrolisierbaren Tanninsäuren, Hydrolyseprodukten der Salze und Ester, Vescalin, Vescalagin, Hydrolyseprodukten von Vescalin oder Vescalagin, Estern und Salzen von Vescalin und Vescalagin, einem Kondensationsprodukt eines Aldehyds mit den Tanninen oder der Tanninsäure, und Gemischen davon.

59. Implantierbarer Träger nach Anspruch 57, wobei die Verbindung, die mindestens einen Ring von 6 Kohlenstoffatomen mit mindestens zwei Hydroxylgruppen daran hat, aus der Gruppe ausgewählt ist, die aus Tanninsäuren mit der folgenden Formel besteht: 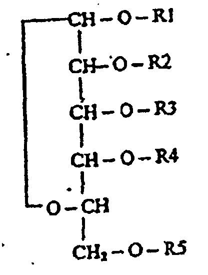 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vacalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

60. Implantierbarer Träger nach einem der Ansprüche 46 bis 56, wobei er an seiner Oberfläche eine Schicht aufweist, die mindestens eine Verbindung enthält, die aus der Gruppe ausgewählt ist, die aus Tanninsäuren mit der folgenden Formel bestehen: 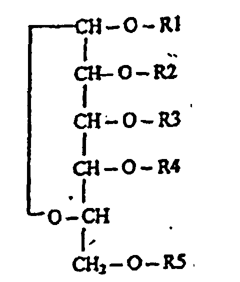 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

61. Implantierbarer Träger nach einem der Ansprüche 46 bis 56, der in Form eines Körpers ist, der an seiner Oberfläche und im Inneren des Körpers eine oder mehrere Verbindungen hat, die aus der Gruppe ausgewählt sind, die aus Tanninsäuren mit der folgenden Formel bestehen: 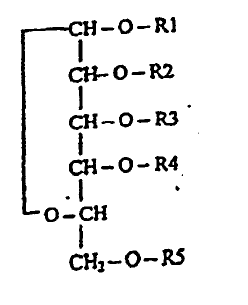 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren; Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, einem Kondensationsprodukt eines Aldehyds mit Tanninen oder Tanninsäure und Gemischen davon.

62. Verwendung einer wäßrigen Zusammensetzung zum Stabilisieren eines implantierbaren Trägers, der aus der Gruppe ausgewählt ist, die besteht aus Trägern, die mit einem biologischen Medium in Kontakt sein sollen, biologischen Trägern, Trägern aus Tiergewebe und Trägern aus Humangewebe, wobei die Zusammensetzung einen Aldehyd im Gemisch mit mindestens einer Verbindung aufweist, die aus der Gruppe ausgewählt ist, die aus Tanninsäuren mit der folgenden Formel besteht: 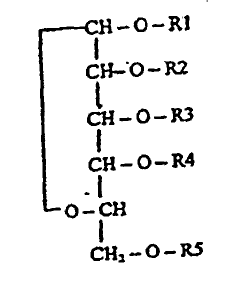 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin, und dem Kondensationsprodukt eines Aldehyds mit Tanninen oder Tanninsäure.

63. Verwendung nach Anspruch 62, wobei die wäßrige Zusammensetzung mindestens eine Verbindung aufweist, die aus der Gruppe ausgewählt ist, die aus Tanninsäuren mit der folgenden Formel besteht: 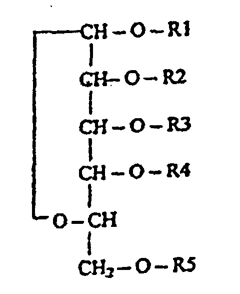 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, Estern und Salzen von Vescalin und Vescalagin im Gemisch mit einem Kondensationsprodukt eines Aldehyds mit Tanninen oder Tanninsäure.

64. Verwendung nach Anspruch 62 oder 63, wobei der pH-Wert der Zusammensetzung zwischen 3 und 9 liegt.

65. Verwendung nach Anspruch 62 oder 63, wobei der pH-Wert der Zusammensetzung zwischen 5,5 und 7,5 liegt.

66. Verwendung nach Anspruch 62 oder 63, wobei der pH-Wert der Zusammensetzung ungefähr 7 ist.

67. Verwendung nach einem der Ansprüche 62 bis 66, wobei die wäßrige Zusammensetzung enthält: bis zu 10 Gew.-% einer ersten Komponente, die aus mindestens einer Verbindung besteht, die aus der Gruppe ausgewählt ist, die aus Tanninsäuren mit der folgenden Formel besteht: 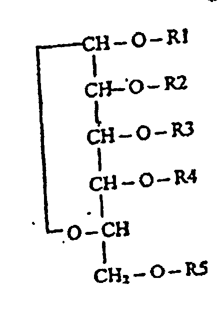 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, und Estern und Salzen von Vescalin und Vescalagin, und bis zu 10 Gew.-% einer zweiten Komponente, die aus einem Aldehyd, einem Kondensationsprodukt eines Aldehyds mit einem Tannin oder einer Tanninsäure oder einem Gemisch davon besteht.

68. Verwendung nach Anspruch 67, wobei die wäßrige Zusammensetzung weniger als 5 Gew.-% der ersten Komponente enthält.

69. Verwendung nach Anspruch 68, wobei die wäßrige Zusammensetzung weniger als 2,5 Gew.-% der ersten Komponente enthält.

70. Verwendung nach einem der Ansprüche 67 bis 69, wobei die wäßrige Zusammensetzung weniger als 5 Gew.-% der zweiten Komponente enthält.

71. Verwendung nach Anspruch 70, wobei die wäßrige Zusammensetzung weniger als 2,5 Gew.-% der zweiten Komponente enthält.

72. Verwendung nach einem der Ansprüche 67 bis 71, wobei das Gewichtsverhältnis der ersten Komponente zu dem Aldehyd zwischen 1:10 und 10:1 ist.

73. Verwendung nach Anspruch 72, wobei das Gewichtsverhältnis der ersten Komponente zu dem Aldehyd zwischen 1:5 und 5:1 ist.

74. Verwendung nach einem der Ansprüche 62 bis 73, wobei die Zusammensetzung einen Phosphatpuffer enthält.

75. Verwendung eines Sets zum Herstellen einer Zusammensetzung zum Stabilisieren eines implantierbaren Trägers nach einem der Ansprüche 62 bis 74, wobei das Set eine erste und eine zweite Flasche aufweist, die Inhalte haben, die zum Herstellen der stabilisierenden Zusammensetzung miteinander zu vermischen sind, wobei:
die erste Flasche als ein Pulver oder in einer wäßrigen Lösung mindestens eine Verbindung enthält, die aus der Gruppe ausgewählt ist, die aus Tanninsäuren mit der folgenden Formel besteht: 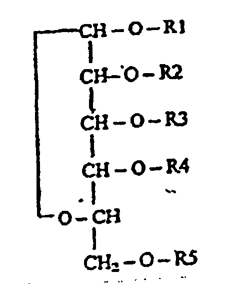 wobei jedes von R¹, R², R³, R⁴ und R⁵ Radikale sind, die erhalten sind nach Entfernen der Hydroxylgruppe von der Carboxylfunktion von Gallussäure oder Digallussäure, Salzen und Estern dieser Tanninsäuren, Chinasäure, Dehydrochinasäure, Estern und Salzen von Chinasäure und von Dehydrochinasäure, Hydrolyseprodukten von Estern und Salzen von Chinasäure und von Dehydrochinasäure, Gallussäure, Digallussäure, Estern und Salzen von Gallussäure und von Digallussäure, Hydrolyseprodukten von Estern und Salzen von Gallussäure und von Digallussäure, Vescalin, Vescalagin, Hydrolyseprodukten von Vescalin oder Vescalagin, und Estern und Salzen von Vescalin und Vescalagin, und die zweite Flasche eine wäßrige Lösung enthält, die einen Aldehyd enthält.

76. Verwendung nach Anspruch 75, wobei die zweite Flasche zusätzlich einen Phosphatpuffer aufweist.

## Revendications

1. Valve cardiaque qui a un support biologique ou biocompatible associé à au moins un composé ayant au moins un cycle de 6 atomes de carbone portant au moins deux groupes hydroxyle.

2. Valve cardiaque selon la revendication 1 dans laquelle le au moins un composé ayant au moins un cycle de 6 atomes de carbone portant au moins deux groupes hydroxyle est présent au moins à la surface du support biologique ou biocompatible.

3. Valve cardiaque selon la revendication 1 ou la revendication 2 dans laquelle le support biologique ou biocompatible est associé à au moins un composé ayant au moins un cycle de 6 atomes de carbone portant au moins trois groupes hydroxyle.

4. Valve cardiaque selon la revendication 1 qui est au moins en partie fabriquée à partir d'un composé polymérique ou copolymérique ou à partir d'un composé biocompatible au moins en partie réticulé associé à au moins un composé ayant au moins un cycle de 6 atomes de carbone portant au moins deux groupes hydroxyle.

5. Valve cardiaque selon la revendication 4 dans laquelle le support biologique ou biocompatible comprend, au moins à sa surface, un ou plusieurs composés ayant au moins un cycle de 6 atomes de carbone portant au moins deux groupes hydroxyle qui sont associés au composé polymérique ou copolymérique ou au composé biocompatible au moins en partie réticulé.

6. Valve cardiaque selon la revendication 4 ou la revendication 5 dans laquelle le composé polymérique ou copolymérique ou le composé biocompatible au moins en partie réticulé est associé à au moins un composé ayant au moins un cycle de 6 atomes de carbone portant au moins trois groupes hydroxyle.

7. Valve cardiaque selon la revendication 1 ou la revendication 2 qui a la forme d'un tissu biologique stabilisé au moins en partie par un composé polymérique ou copolymérique ou par un composé biocompatible au moins en partie réticulé associé à au moins un composé ayant au moins un cycle de 6 atomes de carbone portant au moins deux groupes hydroxyle.

8. Valve cardiaque selon la revendication 7 dans laquelle le composé polymérique ou copolymérique ou le composé biocompatible au moins en partie réticulé est associé à au moins un composé ayant au moins un cycle de 6 atomes de carbone portant au moins trois groupes hydroxyle.

9. Valve cardiaque selon la revendication 7 dans laquelle le tissu biologique est stabilisé au moins en partie par un aldéhyde avec l'aldéhyde à la surface du tissu ou à sa proximité étant au moins en partie associé à un composé ayant au moins un cycle de 6 atomes de carbone portant au moins deux groupes hydroxyle.

10. Valve cardiaque selon la revendication 9 dans laquelle l'aldéhyde à la surface du tissu ou à sa proximité est en moins en partie associé à un composé ayant au moins un cycle de 6 atomes de carbones portant au moins trois groupes hydroxyle.

11. Valve cardiaque selon l'une quelconque des revendications 1 à 10 dans laquelle le composé ayant au moins un cycle de 6 atomes de carbone portant au moins deux groupes hydroxyle est choisi dans le groupe comprenant les tannins, les acides tanniques, les sels d'acides tanniques, les esters d'acides tanniques, les produits d'hydrolyse des sels et des esters d'acides tanniques et des tannins, l'acide quinique, l'acide déhydroquinique, les esters et sels de l'acide quinique et de l'acide déhydroquinique, les produits d'hydrolyse des esters et des sels de l'acide quinique et de l'acide déhydroquinique, l'acide gallique, l'acide digallique, les esters et sels de l'acide gallique et de l'acide digallique, les produits d'hydrolyse des esters et sels de l'acide gallique et de l'acide digallique, l'acide shikimique, l'acide déhydroshikimique, les sels et esters de l'acide shikimique et de l'acide déhydroshikimique, la vescaline, la vescalagine, les produits d'hydrolyse de la vescaline et de la vescalagine, les esters et sels de la vescaline et de la vescalagine, le produit de condensation d'un aldéhyde avec lesdits tannins ou l'acide tannique et leurs mélanges.

12. Valve cardiaque selon la revendication 11 dans laquelle le composé ayant au moins un cycle de 6 atomes de carbone portant au moins deux groupes hydroxyle est choisi dans le groupe comprenant les acides tanniques, les sels d'acides tanniques, les esters d'acides tanniques, les produits d'hydrolyse desdits sels et esters et leurs mélanges.

13. Valve cardiaque selon la revendication 11 dans laquelle le composé ayant au moins un cycle de 6 atomes de carbone portant au moins deux groupes hydroxyle est choisi dans le groupe comprenant les acides tanniques de formule : 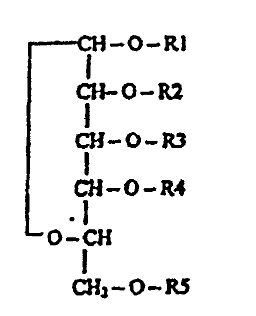 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

14. Valve cardiaque selon l'une quelconque des revendications 2, 5, 7 et 9 qui comprend à sa surface une couche contenant au moins un composé choisi dans le groupe comprenant les acides tanniques de formule : 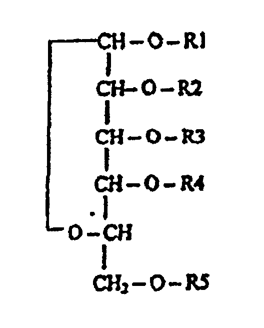 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

15. Valve cardiaque selon l'une quelconque des revendications 1 à 10 qui prend la forme d'un corps ayant à sa surface et à l'intérieur du corps un ou plusieurs composés choisis dans le groupe comprenant les acides tanniques de formule : 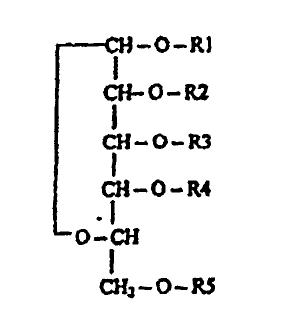 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

16. Utilisation d'un support biocompatible qui est associé à au moins un composé ayant au moins un cycle de 6 atomes de carbone portant au moins deux groupes hydroxyle pour la fabrication d'un implant animal ou humain.

17. Utilisation selon la revendication 16 dans laquelle le support biocompatible est associé à au moins un composé ayant au moins un cycle de 6 atomes de carbone portant au moins trois groupes hydroxyle.

18. Utilisation selon la revendication 16 dans laquelle l'implant a la forme d'un tissu biologique stabilisé au moins en partie par un composé polymérique ou copolymérique ou par un composé biocompatible au moins en partie réticulé associé à au moins un composé ayant au moins un cycle de 6 atomes de carbone portant au moins deux groupes hydroxyle.

19. Utilisation selon la revendication 18 dans laquelle le composé polymérique ou copolymérique ou le composé biocompatible au moins en partie réticulé est associé à au moins un composé ayant au moins un cycle de 6 atomes de carbone portant au moins trois groupes hydroxyle.

20. Utilisation selon la revendication 18 dans laquelle le tissu biologique est stabilisé au moins en partie par un aldéhyde avec l'aldéhyde à la surface du tissu ou à sa proximité étant au moins en partie associé à un composé ayant au moins un cycle de 6 atomes de carbone portant au moins deux groupes hydroxyle.

21. Utilisation selon la revendication 20 dans laquelle l'aldéhyde à la surface du tissu ou à sa proximité est au moins en partie associé à un composé ayant au moins un cycle de 6 atomes de carbone portant au moins trois groupes hydroxyle.

22. Utilisation selon la revendication 16 ou la revendication 17 dans laquelle le support comprend un composé polymérique ou copolymérique ou un composé biocompatible au moins en partie réticulé.

23. Utilisation selon l'une quelconque des revendications 16 à 22 dans laquelle l'implant est une valve cardiaque.

24. Utilisation selon l'une quelconque des revendications 16 à 23 dans laquelle le au moins un composé ayant au moins un cycle de 6 atomes de carbone portant au moins deux groupes hydroxyle est présent au moins à la surface de l'implant.

25. Utilisation selon l'une quelconque des revendications 16 à 24 dans laquelle le composé ayant au moins un cycle de 6 atomes de carbone portant au moins deux groupes hydroxyle est choisi dans le groupe comprenant les tannins, les acides tanniques, les sels d'acides tanniques, les esters d'acides tanniques, les produits d'hydrolyse des sels et esters d'acides tanniques et de tannins, l'acide quinique, l'acide déhydroquinique, les esters et sels d'acide quinique et d'acide déhydroquinique, les produits d'hydrolyse d'esters et sels d'acide quinique et d'acide déhydroquinique, l'acide gallique, l'acide digallique, les esters et sels d'acide gallique et d'acide digallique, les produits d'hydrolyse des esters et sels d'acide gallique et d'acide digallique, l'acide shikimique, l'acide déhydroshikimique, les sels et esters d'acide shikimique et d'acide déhydroshikimique, la vescaline, la vescalagine, les produits d'hydrolyse de la vescaline ou de la vescalagine, les esters et sels de la vescaline et de la vescalagine, le produit de condensation d'un aldéhyde avec lesdits tannins ou l'acide tannique et leurs mélanges.

26. Utilisation selon la revendication 25 dans laquelle le composé ayant au moins un cycle de 6 atomes de carbone portant au moins deux groupes hydroxyle est choisi dans le groupe comprenant les acides tanniques hydrolysables, les sels d'acides tanniques hydrolysables, les esters d'acides tanniques hydrolysables, les produits d'hydrolyse desdits sels et esters, la vescaline, la vescalagine, les produits d'hydrolyse de la vescaline ou de la vescalagine, les esters et sels de la vescaline et de la vescalagine, le produit de condensation d'un aldéhyde avec des tannins ou l'acide tannique et leurs mélanges.

27. Utilisation selon la revendication 25 dans laquelle le composé ayant au moins un cycle de 6 atomes de carbone portant au moins deux groupes hydroxyle est choisi dans le groupe comprenant les acides tanniques de formule : 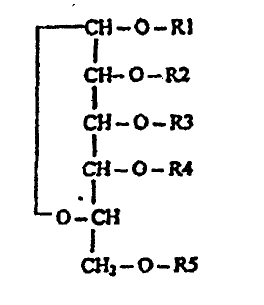 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

28. Utilisation selon l'une quelconque des revendications 16 à 24 dans laquelle l'implant comprend à sa surface une couche contenant au moins un composé choisi dans le groupe comprenant les acides tanniques de formule : 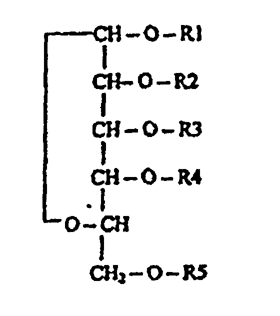 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

29. Utilisation selon l'une quelconque des revendications 16 à 24 dans laquelle l'implant prend la forme d'un corps ayant à sa surface et à l'intérieur du corps un ou plusieurs composés choisis dans le groupe comprenant les acides tanniques de formule : 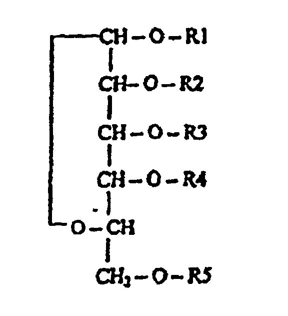 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

30. Méthode pour préparer un implant animal ou humain ayant un support, ladite méthode comprenant les étapes consistant à :
traiter l'implant avec une solution contenant un composé ayant au moins un cycle de 6 atomes de carbone portant au moins deux groupes hydroxyle et
après ledit traitement stériliser ledit implant et/ou traiter ledit implant de manière aseptique.

31. Méthode selon la revendication 30 dans laquelle l'implant est traité avec une solution contenant un composé ayant au moins un cycle de 6 atomes de carbone portant au moins trois groupes hydroxyle.

32. méthode selon la revendication 30 dans laquelle l'implant est au moins en partie préparé à partir d'un composé polymérique ou copolymérique ou à partir d'un composé biocompatible réticulé qui ont été au moins en partie traités avec un composé ayant au moins un cycle de 6 atomes de carbone portant au moins deux groupes hydroxyle.

33. Méthode selon la revendication 32 dans laquelle le composé polymérique ou copolymérique et le composé biocompatible réticulé ont été au moins en partie traités avec un composé ayant au moins un cycle de 6 atomes de carbone portant au moins trois groupes hydroxyle.

34. Méthode selon la revendication 32 ou la revendication 33 dans laquelle le support est associé avec au moins le composé polymérique ou copolymérique ou avec le composé biocompatible en partie réticulé.

35. Méthode selon l'une quelconque des revendications 30 à 34 dans laquelle un tissu biologique qui a été au moins en partie stabilisé par un aldéhyde est utilisé pour l'implant.

36. Méthode selon l'une quelconque des revendications 30 à 35 dans laquelle le composé ayant au moins un cycle de 6 atomes de carbone portant au moins deux groupes hydroxyle est choisi dans le groupe comprenant les tannins, les acides tanniques, les sels d'acides tanniques, les esters d'acides tanniques, les produits d'hydrolyse des sels et esters d'acides tanniques et de tannins, l'acide quinique, l'acide déhydroquinique, les esters et sels d'acide quinique et d'acide déhydroquinique, les produits d'hydrolyse des esters et sels d'acide quinique et d'acide déhydroquinique, l'acide gallique, l'acide digallique, les esters et sels d'acide gallique et d'acide digallique, les produits d'hydrolyse des esters et sels d'acide gallique et d'acide digallique, l'acide shikimique, l'acide déhydroshikimique, les sels et esters d'acide shikimique et d'acide déhydroshikimique, la vescaline, la vescalagine, les produits d'hydrolyse de la vescaline ou de la vescalagine, les esters et sels de la vescaline et de la vescalagine, le produit de condensation d'un aldéhyde avec lesdits tannins ou acides tanniques et leurs mélanges.

37. Méthode selon la revendication 36 dans laquelle le composé ayant au moins un cycle de 6 atomes de carbone portant au moins deux groupes hydroxyle est choisi dans le groupe comprenant les acides tanniques, les sels d'acides tanniques, les esters d'acides tanniques, les produits d'hydrolyse desdits sels et esters et leurs mélanges.

38. Méthode selon l'une quelconque des revendications 30 à 35 dans laquelle le composé ayant au moins un cycle de 6 atomes de carbone portant au moins deux groupes hydroxyle est choisi dans le groupe comprenant les acides tanniques de formule : 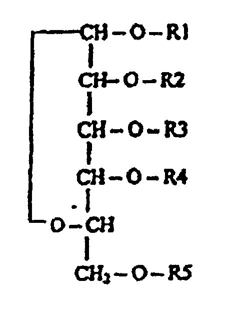 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

39. Méthode selon l'une quelconque des revendications 30 à 39 dans laquelle la solution qui est utilisée pour traiter l'implant a un pH compris entre 3 et 9.

40. Méthode selon l'une quelconque des revendications 30 à 39 dans laquelle la solution qui est utilisée pour traiter l'implant a un pH compris entre 5,5 et 7,5.

41. Utilisation d'au moins un composé choisi dans le groupe comprenant les tannins, les acides tanniques, les sels d'acides tanniques, les esters d'acides tanniques, les produits d'hydrolyse de sels et d'esters d'acides tanniques et de tannins, l'acide quinique, l'acide déhydroquinique, les esters et sels d'acide quinique et d'acide déhydroquinique, les produits d'hydrolyse des esters et sels d'acide quinique et l'acide déhydroquinique, l'acide gallique, l'acide digallique, les esters et sels d'acide gallique et d'acide digallique, les produits d'hydrolyse des esters et sels d'acide gallique et d'acide digallique, l'acide shikimique, l'acide déhydroshikimique, les sels et esters d'acide shikimique et l'acide déhydroshikimique, la vescaline, la vescalagine, les produits d'hydrolyse de la vescaline ou de la vescalagine, les esters et sels de la vescaline et de la vescalagine, le produit de condensation d'un aldéhyde avec lesdits tannins ou acide tannique et leurs mélanges pour la fabrication d'une composition pharmaceutique contenant une quantité efficace dudit au moins un composé pour traiter ou empêcher la calcification dans un circuit sanguin.

42. Utilisation selon la revendication 41 dans laquelle le au moins un composé est choisi dans le groupe comprenant les acides tanniques de formule : 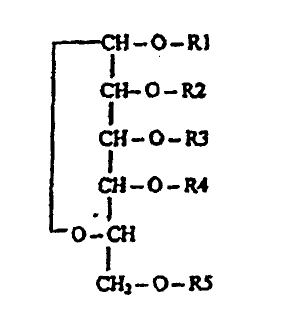 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

43. Utilisation selon la revendication 42 dans laquelle la composition pharmaceutique est utilisée pour traiter ou empêcher la calcification d'une valve cardiaque et/ou d'un implant en contact avec le sang.

44. Utilisation selon la revendication 42 dans laquelle le au moins un composé est choisi dans le groupe comprenant les acides tanniques de formule : 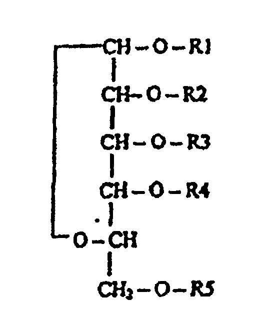 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, des produits d'hydrolyse des sels et esters, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

45. Utilisation selon l'une quelconque des revendications 41 à 44 dans laquelle la composition a la forme d'une composition à libération prolongée.

46. Support implantable pour le contact avec un milieu biologique, ledit support étant associé à au moins un composé ayant au moins un cycle de 6 atomes de carbone portant au moins deux groupes hydroxyle.

47. Support implantable selon la revendication 46 dans lequel le milieu biologique est un humain ou un animal.

48. Support implantable selon la revendication 46 ou la revendication 47 dans lequel le au moins un composé ayant au moins un cycle de 6 atomes de carbone portant au moins deux groupes hydroxyle est présent sur au moins l'une des surfaces du support.

49. Support implantable selon la revendication 48 dans lequel le au moins un composé ayant au moins un cycle de 6 atomes de carbone portant au moins deux groupes hydroxyle est associé à un composé polymérique ou copolymérique ou à un composé biocompatible au moins en partie réticulé.

50. Support implantable comme revendiqué dans l'une quelconque des revendications 46 à 48 comprenant un composé polymérique ou copolymérique et/ou un composé biocompatible au moins en partie réticulé.

51. Support implantable selon l'une quelconque des revendications 46 à 50 dans lequel le support est associé à au moins un composé ayant au moins un cycle de 6 atomes de carbone portant au moins trois groupes hydroxyle.

52. Support implantable selon l'une quelconque des revendications 46 à 48 qui est un support implantable biologique.

53. Support implantable selon l'une quelconque des revendications 46 à 48 dans lequel le support a la forme d'un tissu biologique stabilisé au moins en partie par un composé polymérique ou copolymérique ou par un composé biocompatible au moins en partie réticulé associé à au moins un composé ayant au moins un cycle de 6 atomes de carbone portant au moins deux groupes hydroxyle.

54. Support implantable selon la revendication 53 dans lequel le composé polymérique ou copolymérique ou le composé biocompatible au moins en partie réticulé est associé à au moins un composé ayant au moins un cycle de 6 atomes de carbone portant au moins trois groupes hydroxyle.

55. Support implantable selon la revendication 53 ou la revendication 54 dans lequel le tissu biologique est stabilisé au moins en partie par un aldéhyde, au moins l'aldéhyde qui est à la surface du tissu ou à sa proximité étant au moins en partie associé à un composé ayant au moins un cycle de 6 atomes de carbone portant au moins deux groupes hydroxyle.

56. Support implantable selon la revendication 55 dans lequel l'aldéhyde à la surface du tissu ou à sa proximité est au moins en partie associé à un composé ayant au moins un cycle de 6 atomes de carbone portant au moins trois groupes hydroxyle.

57. Support implantable selon l'une quelconque des revendications 46 à 56 dans lequel le composé ayant au moins un cycle de 6 atomes de carbone portant au moins deux groupes hydroxyle est choisi parmi le groupe comprenant les tannins, les acides tanniques, les sels d'acides tanniques, les esters d'acides tanniques, les produits d'hydrolyse de sels et d'esters d'acides tanniques et de tannins, l'acide quinique, l'acide déhydroquinique, les esters et sels d'acide quinique et d'acide déhydroquinique, les produits d'hydrolyse des esters et sels d'acide quinique et l'acide déhydroquinique, l'acide gallique, l'acide digallique,les esters et sels d'acide gallique et d'acide digallique, les produits d'hydrolyse des esters et sels d'acide gallique et d'acide digallique, l'acide shikimique, l'acide déhydroshikimique, les sels et esters d'acide shikimique et l'acide déhydroshikimique, la vescaline, la vescalagine, les produits d'hydrolyse de la vescaline ou de la vescalagine, les esters et sels de la vescaline et de la vescalagine, le produit de condensation d'un aldéhyde avec lesdits tannins ou acide tannique et leurs mélanges.

58. Support selon la revendication 57 dans lequel le composé ayant au moins un cycle de 6 atomes de carbone portant au moins deux groupes hydroxyle est choisi dans le groupe comprenant les acides tanniques hydrolysables, les sels d'acides tanniques hydrolysables, les esters d'acides tanniques hydrolysables, les produits d'hydrolyse desdits sels et esters, la vescaline, la vescalagine, les produits d'hydrolyse de la vescaline ou de la vescalagine, les esters et sels de la vescaline et de la vescalagine, le produit de condensation d'un aldéhyde avec des tannins ou l'acide tannique et leurs mélanges.

59. Support implantable selon la revendication 57 dans lequel le composé ayant au moins un cycle de 6 atomes de carbone portant au moins deux groupes hydroxyle est choisi dans le groupe comprenant les acides tanniques de formule : 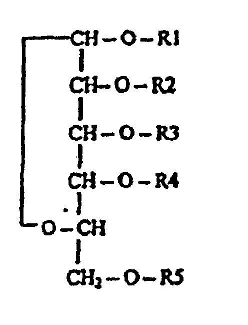 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

60. Support implantable selon l'une quelconque des revendications 46 à 56 qui comprend à sa surface une couche contenant au moins un composé choisi dans le groupe comprenant les acides tanniques de formule : 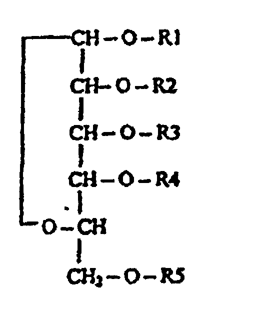 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

61. Support implantable selon l'une quelconque des revendications 46 à 56 qui prend la forme d'un corps ayant à sa surface et à l'intérieur du corps un ou plusieurs composés choisis dans le groupe comprenant les acides tanniques de formule : 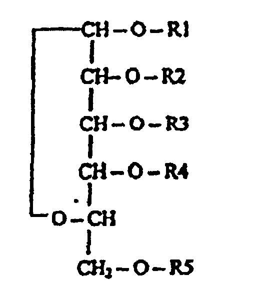 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique et leurs mélanges.

62. Utilisation d'une composition aqueuse pour stabiliser un support implantable choisi dans le groupe des supports conçus pour être en contact avec un milieu biologique, des supports biologiques, des supports de tissu animal et des supports de tissu humain, ladite composition comprenant un aldéhyde en mélange avec au moins un composé choisi dans le groupe comprenant les acides tanniques de formule : 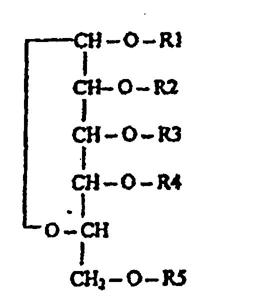 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, du produit de condensation d'un aldéhyde avec des tannins ou l'acide tannique.

63. Utilisation selon la revendication 62 dans laquelle la composition aqueuse comprend au moins un composé choisi dans le groupe comprenant les acides tanniques de formule : 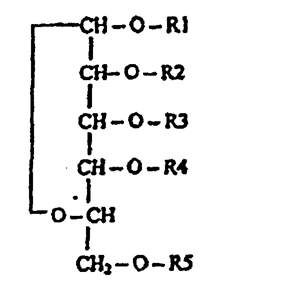 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine, des esters et sels de la vescaline et de la vescalagine, en mélange avec un produit de condensation d'un aldéhyde avec des tannins ou l'acide tannique.

64. Utilisation selon la revendication 62 ou la revendication 63 dans laquelle le pH de la composition est compris entre 3 et 9.

65. Utilisation selon la revendication 62 ou la revendication 63 dans laquelle le pH de la composition est compris entre 5,5 et 7,5.

66. Utilisation selon la revendication 62 ou la revendication 63 dans laquelle le pH de la composition est d'environ 7.

67. Utilisation selon l'une quelconque des revendications 62 à 66 dans laquelle la composition aqueuse contient jusqu'à 10% en poids d'un premier composant consistant en au moins un composé choisi dans le groupe comprenant les acides tanniques de formule : 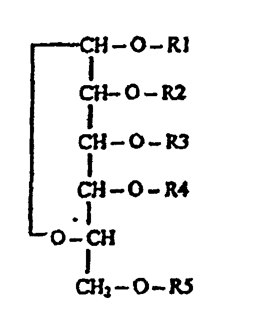 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine et les esters et sels de la vescaline et de la vescalagine et jusqu'à 10% en poids d'un second composant consistant en un aldéhyde, un produit de condensation d'un aldéhyde avec un tannin ou un acide tannique ou l'un de leur mélange.

68. Utilisation selon la revendication 67 dans laquelle la composition aqueuse contient moins de 5% en poids du premier composant.

69. Utilisation selon la revendication 68 dans laquelle la composition aqueuse contient moins de 2,5% en poids du premier composant.

70. Utilisation selon l'une quelconque des revendications 67 à 69 dans laquelle la composition aqueuse contient moins de 5% en poids du second composant.

71. Utilisation selon la revendication 70 dans laquelle la composition aqueuse contient moins de 2,5% en poids du second composant.

72. Utilisation selon l'une quelconque des revendications 67 à 71 dans laquelle le ratio de poids du premier composant par rapport à l'aldéhyde est compris entre 1:10 et 10:1.

73. Utilisation selon la revendication 72 dans laquelle le ratio de poids du premier composant par rapport à l'aldéhyde est compris entre 1:5 et 5:1.

74. Utilisation selon l'une quelconque des revendications 62 à 73 dans laquelle la composition comprend un tampon phosphate.

75. Utilisation d'une trousse pour préparer une composition pour stabiliser un support implantable selon l'une quelconque des revendications 62 à 74, ladite trousse comprenant un premier et un second flacon ayant des contenants à mélanger ensemble pour préparer la composition de stabilisation, dans laquelle :
le premier flacon comprend, sous forme de poudre ou dans une solution aqueuse, au moins un composé choisi dans le groupe comprenant les acides tanniques de formule : 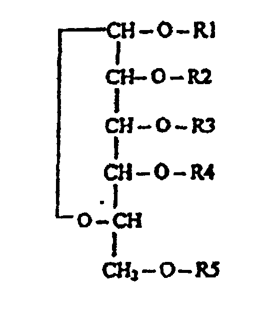 où R¹, R², R³, R⁴ et R⁵ sont des radicaux obtenus après retrait du groupe hydroxyle de la fonction carboxyle de l'acide gallique ou de l'acide digallique, des sels et esters de ces acides tanniques, de l'acide quinique, de l'acide déhydroquinique, des esters et sels de l'acide quinique et de l'acide déhydroquinique, des produits d'hydrolyse des esters et sels de l'acide quinique et de l'acide déhydroquinique, de l'acide gallique, de l'acide digallique, des esters et sels de l'acide gallique et de l'acide digallique, des produits d'hydrolyse des esters et sels de l'acide gallique et de l'acide digallique, de la vescaline, de la vescalagine, des produits d'hydrolyse de la vescaline ou de la vescalagine et des esters et sels de la vescaline et de la vescalagine, et
le second flacon contient une solution aqueuse contenant un aldéhyde.

76. Utilisation selon la revendication 75 dans laquelle le second flacon comprend en outre un tampon phosphate.
